# FASCICULE DE BREVET EUROPEEN

(11) **EP 2 571 528 B1**
(45) Date de publication et mention de la délivrance du brevet: **31.01.2018**
(21) Numéro de dépôt: 11724800.5
(22) Date de dépôt: 18.05.2011
(51) Int. Cl.: A61K 47/54, A61K 47/55, C07C 205/22, C07D 475/04, C07H 15/252, C07H 15/203, A61P 35/00

(54) **BRAS AUTORÉACTIFS ET PRODROGUES LES COMPRENANT**
SELBSTREAKTIVE ARME UND PRODRUGS DAMIT
SELF-REACTIVE ARMS AND PRODRUGS COMPRISING SAME

(30) Priorité: 20.05.2010 FR 1053921
(43) Date de publication de la demande: 27.03.2013
(73) Titulaire: Centre National de la Recherche Scientifique, 75794 Paris Cedex 16 (FR); Université De Poitiers, 86034 Poitiers Cedex (FR)
(72) Inventeur: PAPOT, Sébastien, 86000 Poitiers (FR); THOMAS, Mickaël, 86110 Varennes (FR)
(74) Mandataire: Lavoix
(86) Numéro de dépôt international: PCT/IB2011/052184
(87) Numéro de publication internationale: WO 2011/145068

(56) Documents cités:
- EP-A1- 0 511 917
- EP-A1- 0 642 799
- WO-A2-2005/115105
- GOPIN A ET AL: "A CHEMICAL ADAPTOR SYSTEM DESIGNED TO LINK A TUMOR-TARGETING DEVICE WITH A PRODRUG AND AN ENZYMATIC TRIGGER", ANGEWANDTE CHEMIE. INTERNATIONAL EDITION, WILEY VCH VERLAG, WEINHEIM, vol. 42, no. 3, 20 janvier 2003 (2003-01-20), pages 327-332, XP001197453, ISSN: 1433-7851, DOI: DOI:10.1002/ANIE.200390108 cité dans la demande
- KOLB H C ET AL: "The growing impact of click chemistry on drug discovery", DRUG DISCOVERY TODAY, ELSEVIER, RAHWAY, NJ, US, vol. 8, no. 24, 15 décembre 2003 (2003-12-15), pages 1128-1137, XP002377521, ISSN: 1359-6446, DOI: DOI:10.1016/S1359-6446(03)02933-7
- SHIHUI WU ET AL: "Reaction of Propargyl Bromide with Aldehydes in the Presence of Metallic Tinhesis of Homopropargylalcohols and Homoallenylalcohols", SYNTHETIC COMMUNICATIONS, TAYLOR & FRANCIS GROUP, PHILADELPHIA, PA, vol. 20, no. 9, 1 janvier 1990 (1990-01-01), pages 1279-1286, XP008129135, ISSN: 0039-7911
- MATSUDA H ET AL: "Structure-activity relationships of 1'S-1'-acetoxychavicol acetate for inhibitory effect on NO production in lipopolysaccharide-activated mouse peritoneal macrophages", BIOORGANIC & MEDICINAL CHEMISTRY LETTERS, PERGAMON, ELSEVIER SCIENCE, GB, vol. 15, no. 7, 1 avril 2005 (2005-04-01), pages 1949-1953, XP025313482, ISSN: 0960-894X, DOI: DOI:10.1016/J.BMCL.2005.01.070 [extrait le 2005-04-01]
- Hajime Ito ET AL: "Intramolecular hydrogen bond-promoted C-C bond formation: reaction rate enhancement and regioselective allylation of carbonyl compounds", Chemical Communications, 1 January 1998 (1998-01-01), pages 2443-2444, XP055288684, Retrieved from the Internet: URL:http://pubs.rsc.org/en/content/article pdf/1998/cc/a807172a [retrieved on 2016-07-14]
- M. Arjun Reddy ET AL: "Asymmetric synthesis of 2-azido-1-arylethanols from azido aryl ketone-[beta]-cyclodextrin complexes and sodium borohydride in water", CHEMICAL COMMUNICATIONS - CHEMCOM., no. 19, 1 January 2001 (2001-01-01), pages 1974-1975, XP055288862, ISSN: 1359-7345, DOI: 10.1039/b106736m

## Description

La présente invention se rapporte au domaine des prodrogues et à leur préparation. Plus particulièrement, la présente invention concerne un nouveau bras auto-immolable ou auto-réactif et son utilisation pour la préparation de nouvelles prodrogues. La présente invention concerne également de nouvelles prodrogues comprenant un tel bras et permettant l'adressage spécifique de composés actifs *in vivo.* La présente invention concerne également un procédé de préparation de nouvelles prodrogues mettant en oeuvre un nouveau bras auto-immolable ou auto-réactif, ainsi que des compositions pharmaceutiques ou diagnostiques comprenant ces prodrogues.

Un composé actif administré à un être vivant, par exemple à visée thérapeutique ou diagnostique, est caractérisé par un profil d'absorption, de distribution, de métabolisation et d'élimination. L'ensemble des paramètres de ce profil participe à la définition de sa biodisponibilité et de ses effets thérapeutiques ou diagnostiques.

Selon les caractéristiques de ce profil, un composé actif pourra exercer un effet thérapeutique ou diagnostique de manière plus ou moins soutenue dans le temps, de façon plus ou moins intense, et pourra être associé, le cas échéant, à des effets secondaires plus ou moins marqués. La forme selon laquelle ce composé actif est administré peut s'avérer déterminante à l'égard de son profil d'activité et de sa toxicité.

De nombreux composés actifs, par exemple les actifs anticancéreux, présentent l'inconvénient d'être toxiques à l'égard des tissus sains ou des cellules saines, limitant ainsi leur utilisation.

Le cancer est aujourd'hui une des premières causes de mortalité en France. Parmi les différents modes de traitements envisageables, la chimiothérapie est la seule utilisable à l'encontre des tumeurs circulantes, telles que les lymphomes et les leucémies, et les métastases. Toutefois, les actifs anticancéreux utilisés cliniquement présentent peu de sélectivité vis-à-vis des cellules tumorales et s'attaquent également aux tissus sains. Cette destruction non-sélective entraîne de sévères effets secondaires et conduit dans la plupart des cas à l'arrêt prématuré du traitement. Le développement de nouveaux agents anticancéreux conçus pour détruire sélectivement les tumeurs sans affecter les organes sains représente donc un intérêt majeur dans la lutte contre le cancer.

Des études récentes ont mis en évidence que les tissus tumoraux pourraient être différenciés des tissus sains d'une part à l'égard du microenvironnement tumoral qui se distingue des tissus sains par un pH plus acide, un potentiel réducteur plus important, une perméabilité accrue pour les macromolécules ou encore par la présence d'une concentration relativement élevée de certaines enzymes, telles que la β-glucuronidase, et d'autre part à l'égard des cellules malignes qui surexpriment à leur surface des récepteurs membranaires ou des antigènes qui les différencient des cellules saines, telles que les récepteurs de l'acide folique ou l'antigène CD33.

Afin de réduire la toxicité de composés actifs tels que les actifs anticancéreux, ou d'améliorer leur biodisponibilité, *via* par exemple une meilleure solubilisation, et leur adressage vers les tissus et cellules cibles, il a été proposé de les vectoriser sous forme de prodrogues. Ces composés sont ainsi, le plus souvent, rendus inertes par greffages chimiques sur une molécule destinée à les véhiculer, au sein d'un organisme dans lequel ils sont administrés, jusqu'aux tissus ou cellules cibles, où ils seront libérés.

Dans ce but, de nombreuses molécules à fonction de bras, ou espaceur, auto-immolable, ou auto-réactif, ont été proposées afin de vectoriser des composés actifs sous une forme inerte, de manière spécifique et ciblée.

Le bras auto-réactif est lié de manière covalente, d'une part au composé actif à vectoriser, et d'autre part à un groupe labile permettant d'assurer une spécificité cellulaire ou tissulaire et/ou à une solubilisation améliorée dans les fluides biologiques. L'élimination du groupe labile conduit, par réarrangement intramoléculaire du bras auto-réactif, à la libération du composé actif.

Un bras auto-réactif se doit de conférer à la prodrogue une stabilité suffisante permettant son administration, par exemple par voie orale ou systémique et doit être doté d'une réactivité suffisante pour permettre la libération du composé actif de manière immédiate ou quasi-immédiate consécutivement à l'élimination du groupe labile.

Tranoy-Opalinski et al. (Anti-Cancer Agent Med. Chem., 2008, 8:1) décrivent différents types de bras auto-réactifs convenant à la préparation de prodrogues. En particulier, sont décrits des prodrogues comprenant à titre de bras auto-réactifs un dérivé du phénol ou de l'aniline, conduisant à une élimination 1,6, et dans lesquels le composé actif est attaché sur un carbone benzylique au moyen d'une fonction carbamate ou carbonate et la fonction phénol est liée à un groupe glucuronyle éliminable sous l'action d'une β-glucuronidase. La présence du groupe glucuronide permet d'assurer la spécificité tissulaire des prodrogues vers les tumeurs au sein desquelles la β-glucuronidase est fortement exprimée.

Toutefois, les prodrogues dont la spécificité tissulaire est assurée par un groupe labile substrat d'une enzyme présentent l'inconvénient de nécessiter, généralement, que l'enzyme assurant l'hydrolyse de ce groupe soit au préalable adressée vers le tissu ou l'organe ciblée. Ainsi, WO 81/01145, EP 0 642 799 ou EP 0 511 917 décrivent la mise en oeuvre de tels prodrogues dans des thérapies par prodrogues activables par des enzymes adressées par des anticorps (ou « antibody directed enzyme prodrogue therapy », ADEPT).

Par ailleurs, Jeffrey et al. (Bioorg. Med., Chem., Lett., 2007, 17 : 2278) décrivent une prodrogue comprenant un bras auto-réactif dérivé du phénol sur lequels sont greffés un groupe glucuronyle sur la fonction phénol, un composé actif sur un carbone benzylique par une fonction carbamate, et en ortho de la fonction phénol, un anticorps par une fonction amido. L'adressage spécifique du composé actif est assuré par l'anticorps, et l'hydrolyse du groupe glucuronyle par action d'une β-glucuronidase intracellulaire assure la libération du composé actif dans les cellules cibles. Toutefois, les anticorps sont des macromolécules pénétrant de manière peu efficace dans la zone tumorale, et ne sont éliminés que très lentement par l'organisme.

On peut également, citer Gopin et al. (Angew. Chem. Inter. Ed., 2003, 42: 327) qui décrivent une prodrogue comprenant un composé actif lié à un bras auto-réactif dérivé de l'acide 4-hydroxymandélique, sur lequel sont fixés d'une part un groupe substrat de l'anticorps catalytique 38C2, et un ligand de ciblage, le HPMA. La libération du composé actif à partir de cette prodrogue s'avère toutefois être relativement lente.

Enfin, on peut citer EP 2 098 534 qui décrit une prodrogue comprenant une anthracycline lié un bras auto-réactif de type *para* aminobenzylcarbonyle par une fonction carbamate sur le carbone benzylique, et un groupe glucuronyl (ou gâchette glucuronylée) lié par une fonction carbamate au phényl. Le groupe glucuronyl est en outre fonctionnalisé par un groupe propargyle permettant par chimie click la fixation d'un radical polyéthylèneglycol à fin d'améliorer l'hydrosolubilité de l'ensemble.

Le développement de prodrogues aptes à vectoriser de manière stable, spécifique et en grandes quantités des composés actifs sous une forme inerte et de libérer ces derniers rapidement et de façon localisée s'avère être de toute première importance vis-à-vis de composés actifs dont la fenêtre thérapeutique est étroite, comme par exemple les actifs anticancéreux.

Ainsi, il existe un besoin de disposer d'un bras auto-réactif permettant l'obtention de manière simple et rapide de prodrogues aptes à vectoriser avec une très grande spécificité et sous forme inerte des composés actifs à fenêtre thérapeutique étroite, notamment des actifs anticancéreux.

Il existe un besoin de disposer d'un bras auto-réactif sur lequel peuvent être greffés chimiquement de manière simple et avec un grand rendement une grande variété de composés actifs et de groupe permettant d'assurer une spécificité tissulaire et cellulaire et/ou une solubilisation améliorée dans les fluides biologiques.

Il existe encore un besoin de disposer d'un bras auto-réactif permettant la préparation de prodrogues dotées d'une grande stabilité convenant à une administration par voie orale ou dans le flux sanguin, et permettant une libération rapide, voire immédiate ou quasi-immédiate du composé actif au niveau du tissu ou de la cellule ciblée.

Il existe encore un besoin de disposer d'un bras auto-réactif convenant à la préparation de prodrogues permettant d'adresser à la fois sur le plan tissulaire et sur le plan cellulaire un composé actif, notamment un actif anticancéreux.

Il existe également un besoin de disposer de nouvelles prodrogues qui puissent cibler simultanément le microenvironnement tissulaire et les cellules du tissu ou de l'organe visé.

Il existe également un besoin de disposer de prodrogues qui puissent être préparées de manière simple et avec un rendement convenant à une production industrielle.

Il existe encore un besoin de disposer de prodrogues dotées d'une bonne stabilité, notamment dans le flux sanguin, et permettant la libération immédiate ou quasi immédiate du composé actif, notamment un actif anticancéreux dans le tissu ciblé sous l'action d'une enzyme ou d'un changement de conditions environnementales, telles qu'une variation de pH.

Il existe également un besoin de disposer de nouvelles prodrogues dont les paramètres de ciblage, à la fois de l'environnement tissulaire et des cellules, ainsi que les paramètres de solubilité peuvent être aisément et finement modulés.

Enfin, il existe encore un besoin de disposer d'une prodrogue pouvant être administrée en une quantité compatible avec une utilisation thérapeutique ou diagnostique et permettant d'adresser une quantité efficace de composés actifs dans le ou les tissus ou cellules ciblées.

La présente invention a pour objet de satisfaire ces besoins. La présente demande divulgue un composé de formule générale (I) : 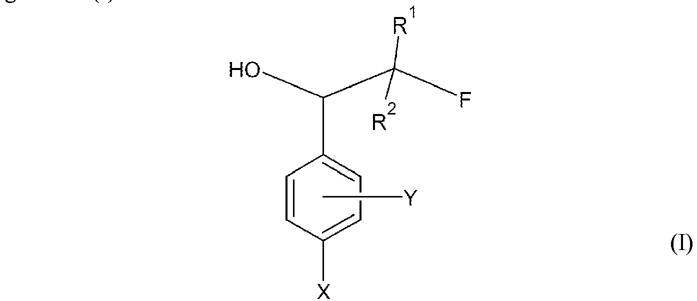 dans laquelle :
- X peut représenter OH, NH₂, NHOH ou R'NH avec R' pouvant représenter un radical alkyle en C₁ à C₁₀, linéaire ou ramifié, saturé ou insaturé,
- Y peut représenter H, ou un groupe électro-attracteur, en particulier choisi parmi NO₂, CF₃ ou un halogène,
- R¹ et R² peuvent représenter, indépendamment l'un de l'autre, H ou un radical alkyle en C₁ à C₁₀, linéaire ou ramifié, saturé ou insaturé,
- F peut représenter une fonction réactive activable par chimie click, en particulier choisie parmi -C≡CR"', -N₃, -SH, -C=CH₂, cyclooctynes, maléimide, -SO₂N₃, ou - COSR"', avec R'" représentant H ou un radical alkyle, linéaire ou ramifié, saturé ou insaturé, en C₁ à C₁₀.
Selon un premier aspect, la présente invention concerne un composé de formule générale (I) dans laquelle Y est un groupe électro-attracteur choisi parmi NO₂, CF₃ ou un halogène, tel que défini dans la revendication 1. De manière surprenante, les inventeurs ont observé qu'il était possible, en partant d'un bras auto-réactif dérivé du phénol, et plus particulièrement du type *para-*hydroxybenzylcarbonyle, d'introduire sur le carbone benzylique une fonction réactive convenant à la chimie click, notamment une fonction de type alcyne et en particulier éthynyle, sous réserve qu'elle soit liée au carbone benzylique, directement ou indirectement par l'intermédiaire d'un carbone sp³. Les inventeurs ont également observé que la cinétique de libération du composé actif pouvait être modulée, et notamment être accélérée en faisant varier la nature du substituant sur l'une des positions en *ortho* ou *meta* par rapport au carbone benzylique, et de préférence en position *meta.*

Les inventeurs ont ainsi développé un nouveau bras auto-réactif sur lesquels peuvent être greffés par des réactions chimiques simple un composé actif, notamment thérapeutique ou diagnostique, et en particulier un actif anticancéreux, un ligand de ciblage, en particulier un groupe issu d'un acide folique, et un groupe labile, notamment par hydrolyse enzymatique, en particulier un groupe glucuronyle.

De manière surprenante, un bras auto-réactif de l'invention peut être mis à profit de manière simple pour la préparation de prodrogues permettant de vectoriser de manière inerte un composé actif, notamment un actif anticancéreux, et d'assurer un adressage à la fois au regard de spécificités du microenvironnement tissulaire et au regard de spécificités cellulaires.

Les inventeurs ont pu en outre observer de manière surprenante que les prodrogues préparées au moyen d'un bras auto-réactif selon l'invention s'avéraient être particulièrement stables dans des conditions physiologiques, et qu'elles étaient aptes à libérer de manière spécifique et très rapidement une quantité efficace de composés actifs.

Les prodrogues de l'invention comportent quatre unités distinctes, chacune conçue pour exercer une fonction particulière.

Une première unité est constituée d'un composé actif, notamment responsable d'une activité thérapeutique ou diagnostique, par exemple un actif anticancéreux. Le composé actif est rendu inerte par fixation sur le bras auto-réactif de l'invention de sorte que son éventuelle toxicité est avantageusement masquée afin de ne pas affecter les tissus sains.

Une seconde unité est constituée d'un groupe labile, notamment enzymatiquement hydrolysable, figurant une gâchette (ou trigger) et qui assure la reconnaissance d'une cible, notamment une enzyme, sélectivement localisée dans le microenvironnement tissulaire visé ou dans un type de cellules spécifique, et dont l'hydrolyse déclenche la libération du composé actif.

Une troisième unité est constituée du ligand de ciblage (ou « targeting ligand ») qui reconnaît un récepteur membranaire exprimé spécifiquement à la surface des cellules du tissus visé et qui permet l'internalisation de la prodrogue par le phénomène d'endocytose.

Enfin, une quatrième unité est constituée du bras auto-réactif de l'invention qui assure la cohésion et la stabilité de la prodrogue dans les conditions physiologiques et l'expulsion de composé actif exclusivement après élimination du groupe labile.

Un composé de formule générale (I) conforme à l'invention constitue un bras réactif auto-réactif dans lequel F, X et -OH sur le carbone benzylique constitue des fonctions réactives susceptibles d'être mobilisées de manière simple et spécifique et sur lesquelles peuvent être greffés différents composés de sorte à former ensemble avec le bras réactif auto-réactif de l'invention une prodrogue selon l'invention.

Ainsi, selon un deuxième objet, l'invention concerne un composé de formule générale (II) : 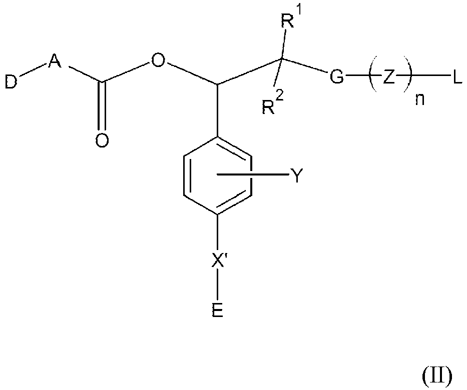 dans laquelle :
- Y peut représenter H, ou un groupe électro-attracteur, en particulier choisi parmi NO₂, CF₃ ou un halogène, R¹ et R² sont tels que définis précédemment,
- X' peut représenter O, NH, NOH, R'N avec R' étant tel que défini précédemment,
- E peut représenter un groupe labile substrat d'une enzyme choisie parmi les glucuronidases, les glycosidases, les protéases, les peptidases, et les métalloprotéases,
- A peut représenter O, S, NH, NR" avec R" représentant un groupe alkyle en C₁ à C₁₀, linéaire ou ramifié, saturé ou insaturé, et de préférence peut représenter NH,
- D peut représenter un composé actif utilisable en thérapeutique ou en diagnostique,
- n = 0 ou 1, et Z peut représenter un groupe alkylène, linéaire ou ramifié, saturé ou insaturé, en C₁-C₁₀, optionnellement interrompu par un ou plusieurs hétéroatomes choisis parmi O ou N, un groupe glycosyle, un groupe O-(CHR³-CHR⁴-O-)ₘ ou N-(CHR³-CHR⁴-O-)ₘ dans lesquels m figure un entier naturel variant de 1 à 20, R³ et R⁴ figurent, indépendamment l'un de l'autre, H ou CH₃, sous réserve que R³ et R⁴ ne figurent pas simultanément CH₃, un groupe issu d'un acide aminé ou d'un peptide, ou une combinaison de ces groupes,
- L peut représenter un ligand de ciblage choisi parmi un peptide, une protéine, un anticorps ou un fragment d'anticorps reconnaissant un antigène, un ligand d'un récepteur cellulaire, un biopolymère, un monosaccharide, un oligosaccharide, une hormone, une vitamine, un dendrimère, une polyamine, ou une nanoparticule,
- G représente un groupe résultant d'une réaction de chimie click entre un groupe F, tel que défini précédemment et un groupe x-(Z)ₙ-L dans lequel Z et L sont tels que définis ci-dessus et x représente une fonction réactive activable par chimie click apte à réagir avec F, ou un de ses sels pharmaceutiquement acceptables.

Selon encore un autre objet, la présente invention concerne une utilisation d'un composé de formule générale (I) pour la préparation de prodrogue, en particulier figurée par les composés de formule générale (II) définis ci-après.

Selon encore un autre objet, la présente invention concerne un procédé de préparation d'un composé de formule générale (II) tel que défini précédemment, ou un de ses sels pharmaceutiquement acceptables, consistant à faire réagir un composé de formule générale (I) tel que défini précédemment avec des groupes D-v, E-w, et L-(Z)ₙ-x, dans lesquels D, E et L peuvent être tels que définis précédemment, et v, w et x figurent chacun une fonction réactive telle que, à l'égard du composé de formule générale (I), v réagisse avec OH lié au carbone benzylique, ou ledit OH préalablement activé, w réagisse X, et x réagisse avec F.

Les fonctions réactives v, w et x peuvent être naturellement présentent sur les groupes D, E et L-(Z)ₙ, ou peuvent être, préalablement à l'étape de réaction, introduites sur les groupes D, E et L-(Z)ₙ.

Selon encore un autre objet, la présente invention concerne un composé ou un de ses sels pharmaceutiquement acceptables, susceptible d'être obtenu selon un procédé de l'invention.

Selon encore un autre aspect, l'invention concerne une composition pharmaceutique ou diagnostique comprenant au moins une quantité efficace d'au moins un composé de formule générale (II) conforme à l'invention, ou un de ses sels pharmaceutiquement acceptables.

Selon encore un autre objet, la présente invention concerne un kit pour la préparation d'un composé de formule générale (II) tel que défini précédemment, ou de ses sels pharmaceutiquement acceptables, comprenant au moins un composé de formule générale (I) tel que défini précédemment, et au moins un groupe choisi parmi D-v, E-w et L-x, dans lesquels L, D et E sont tels que définis précédemment et v, w et x sont tels que définis en précédemment.

Selon un mode de réalisation, un kit selon l'invention peut en outre comprendre au moins une instruction de mise en oeuvre du composé de formule générale (I) avec ledit groupe choisi parmi D-v, E-w et L-x.

Au sens de l'invention, on entend par « bras auto-réactif » un composé apte à libérer par réarrangement intra-moléculaire des groupes préalablement greffés sur celui-ci.

Au sens de l'invention, on entend par « prodrogue » une molécule apte à véhiculer sous forme inerte un composé actif au sein d'un organisme, et de libérer celui-ci dans un organe, un tissu ou des cellules spécifiquement ciblées sous l'action, par exemple d'une enzyme spécifique ou de conditions de pH particulières.

Un bras auto-réactif selon l'invention peut avantageusement permettre de fixer de manière simple et spécifique un composé actif et deux groupes destinés à assurer le ciblage du composé actif et/ou améliorer sa solubilité dans un environnement biologique.

Selon un autre avantage, un bras auto-réactif selon l'invention est doté de fonctions réactives permettant l'obtention de manière simple et en quantités importantes de prodrogues.

Selon un autre avantage, un bras auto-réactif selon l'invention permet la préparation de prodrogues stables, aptes à véhiculer un composé actif sous forme inerte, dotées d'une double spécificité tissulaire et cellulaire, et aptes à libérer de manière ciblée, efficace et rapide ledit composé actif.

Selon un autre avantage, la présente invention permet de disposer de prodrogues, susceptibles d'être utilisées en diagnostique ou en thérapeutique, et permettant le transport ciblé sous forme inerte d'un composé actif, et son adressage de manière spécifique à la fois à l'égard du microenvironnement tissulaire et à l'égard de récepteurs ou de protéines présents à la surface des cellules visées.

Selon un autre avantage, la présente invention permet de disposer de prodrogues stables, dotées d'une double spécificité tissulaire et cellulaire, et activables à l'intérieur de cellules spécifiquement ciblées.

Selon un autre avantage, la présente invention permet de disposer de prodrogues stables et activables dans l'environnement tissulaire du tissu cible au moyen d'enzymes spécifiques présentes au sein de ce tissu ou préalablement adressées par couplage avec un anticorps.

Selon un autre avantage, la présente invention permet de disposer de prodrogues dont la solubilité peut être aisément contrôlée et améliorée.

Selon encore un autre avantage, un bras auto-réactif de l'invention convient particulièrement à la préparation de prodrogues destinées à vectoriser des composés anticancéreux.

### Bras auto-réactif

Un bras auto-réactif selon l'invention peut être figuré par la formule générale (I) suivante : 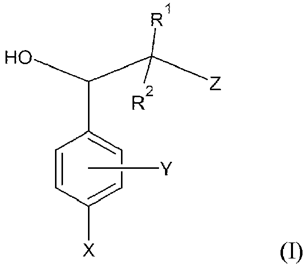 dans laquelle :
- X peut représenter OH, NH₂, NHOH ou R'NH avec R' pouvant représenter un radical alkyle en C₁ à C₁₀, linéaire ou ramifié, saturé ou insaturé,
- Y peut représenter un groupe électro-attracteur, choisi parmi NO₂, CF₃ ou un halogène,
- R¹ et R² peuvent représenter, indépendamment l'un de l'autre, H ou un radical alkyle en C₁ à C₁₀, linéaire ou ramifié, saturé ou insaturé,
- F peut représenter une fonction réactive activable par chimie click.

Selon un mode de réalisation préféré, X peut représenter OH, NH₂, NH(OH) ou R'NH avec R' pouvant représenter un radical alkyle linéaire ou ramifié, saturé ou insaturé, en C₂ à C₈, de préférence en C₃ en C₆, et de préférence en C₄ ou C₅. De manière préférée encore R' peut représenter un radical alkyle en C₁, C₂ ou C₃.

De manière plus préférée encore, X peut représenter OH.

Selon un mode de réalisation, Y peut figurer NO₂, un halogène choisi parmi Cl ou Br, ou CF₃. En particulier, Y peut figurer NO₂ ou Cl, et de préférence représente NO₂.

De manière préférée, Y peut être en *ortho* de X.

Selon une variante de réalisation encore plus préférée, Y peut figurer NO₂ en position *ortho* par rapport à X.

Selon un mode de réalisation, R¹ et R² peuvent représenter, indépendamment l'un de l'autre, H ou un radical alkyle en C₁ à C₁₀, linéaire ou ramifié, saturé ou insaturé, de préférence en C₂ à C₈, de préférence en C₃ en C₆, et de préférence en C₄ ou C₅. De manière préférée encore R¹ ou R² peuvent représenter un radical alkyle en C₁, C₂ ou C₃.

Selon une variante de réalisation, R¹ peut représenter un groupe alkyle, notamment un méthyle, un éthyle ou un propyle, et R² peut représenter H. Selon un autre mode de réalisation, R¹ peut représenter H et R² peut représenter un groupe alkyle, notamment un méthyle, un éthyle ou un propyle.

Selon une variante de réalisation préférée, R¹ et R² figurent chacun H.

F représente une fonction réactive activable par chimie click. La chimie click est processus réactionnel connu de l'homme de l'art. A cet égard on peut se référer à la revue de Kolb et al. (Angew. Chem. Int. Ed., 2001, 40: 2004).

Selon un mode de réalisation, F représente une fonction réactive activable par chimie click choisie parmi F représente -C≡CR"', -N₃, -SH, -C=CH₂, cyclooctynes, maléimide, -SO₂N₃, ou -COSR"', avec R'" représentant H ou un radical alkyle, linéaire ou ramifié, saturé ou insaturé, en C₁ à C₁₀.

Selon un mode de réalisation préféré, F représente une fonction réactive activable par chimie click choisie parmi -C≡CH, -N₃, -SH, -C=CH₂, cyclooctynes, maléimide, -SO₂N₃, ou -COSR.

Selon un mode de réalisation particulièrement préféré, un bras auto-réactif convenant à l'invention peut être de formule (Ia) suivante : 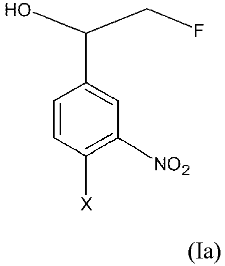 dans laquelle X et F sont tels que définis précédemment.

De manière plus préférée encore, un bras auto-réactif convenant à l'invention peut être de formule (Ia) dans laquelle X représente OH, et F est tel que défini précédemment.

Selon un autre mode de réalisation particulièrement préféré, un bras auto-réactif convenant à l'invention peut être de formule (Ib) suivante :

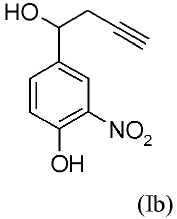

Un bras auto-réactif convenant à l'invention peut, par exemple, être obtenu à partir de 4-hydroxy-3-nitro benzaldéhyde ou de 4-amino benzaldéhyde mis à réagir dans un solvant approprié et en présence d'éventuel(s) catalyseur(s) avec tout réactif apte à introduire sur le carbone benzylique une fonction réactive propice à la réalisation de réactions ultérieures par chimie click.

Selon un mode de réalisation particulier, un bras auto-réactif selon l'invention comprenant à titre de fonction réactive F un groupe -C=CH et à titre de groupe X une fonction OH peut être obtenu par un procédé comprenant une étape de réaction entre du 4-hydroxy-3-nitro benzaldéhyde et l'organo-aluminique du bromure de propargyle, dans un solvant appropriée.

Un solvant convenant à un tel procédé peut être par exemple le tétrahydrofurane.

Un tel procédé peut être effectué en présence de catalyseur. Un catalyseur convenant à l'invention peut, par exemple, être le chlorure de mercure (HgCl₂).

Selon une variante de réalisation, un tel procédé peut comprendre une étape de chauffage, par exemple à reflux, du 4-hydroxy-3-nitro benzaldéhyde en présence du catalyseur, notamment le chlorure de mercure (HgCl₂).

Selon, une variante de réalisation, un tel procédé peut comprendre une étape d'hydrolyse du produit obtenu après réaction du 4-hydroxy-3-nitro benzaldéhyde avec le bromure de propargyle en présence de HCl.

Un procédé selon l'invention peut en outre comprendre une étape consistant à isoler et/ou à purifier le produit obtenu. Les étapes consistant à isoler et/ou purifier peuvent être effectuées selon toutes techniques connues de l'homme du métier.

### Prodrogues

Une prodrogue selon l'invention peut être de formule générale (II) suivante : 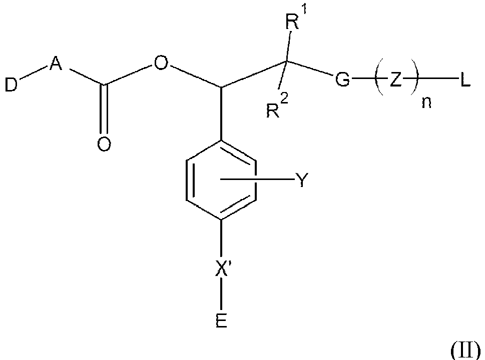 dans laquelle :
- Y peut représenter H, ou un groupe électro-attracteur, en particulier choisi parmi NO₂, CF₃ ou un halogène, R¹ et R² sont tels que définis précédemment,
- X' peut représenter O, NH, NOH, R'N avec R' étant tel que défini précédemment,
- E peut représenter un groupe labile substrat d'une enzyme choisie parmi les glucuronidases, les glycosidases, les protéases, les peptidases, et les métalloprotéases,
- A peut représenter O, S, NH, NR" avec R" représentant un groupe alkyle en C₁ à C₁₀, linéaire ou ramifié, saturé ou insaturé, et de préférence peut représenter NH,
- D peut représenter un composé actif utilisable en thérapeutique ou en diagnostique,
- n = 0 ou 1, et Z peut représenter un groupe alkylène, linéaire ou ramifié, saturé ou insaturé, en C₁-C₁₀, optionnellement interrompu par un ou plusieurs hétéroatomes choisis parmi O ou N, un groupe glycosyle, un groupe O-(CHR³-CHR⁴-O-)ₘ ou N-(CHR³-CHR⁴-O-)ₘ dans lesquels m figure un entier naturel variant de 1 à 20, R³ et R⁴ figurent, indépendamment l'un de l'autre, H ou CH₃, sous réserve que R³ et R⁴ ne figurent pas simultanément CH₃, un groupe issu d'un acide aminé ou d'un peptide, ou une combinaison de ces groupes,
- L peut représenter un ligand de ciblage choisi parmi un peptide, une protéine, un anticorps ou un fragment d'anticorps reconnaissant un antigène, un ligand d'un récepteur cellulaire, un biopolymère, un monosaccharide, un oligosaccharide, une hormone, une vitamine, un dendrimère, une polyamine, ou une nanoparticule,
- G représente un groupe résultant d'une réaction de chimie click entre un groupe F, tel que défini précédemment et un groupe x-(Z)ₙ-L dans lequel Z et L sont tels que définis ci-dessus et x représente une fonction réactive activable par chimie click apte à réagir avec F, ou un de ses sels pharmaceutiquement acceptables.

Selon un mode préféré de réalisation, une prodrogue de l'invention peut être de formule générale (II), dans laquelle Y peut représenter NO₂ en position *ortho* de X', et R¹ et R² peuvent représentent H.

Un sel pharmaceutiquement acceptable d'un composé de formule générale (II) selon l'invention peut être un sel d'un composé de formule générale (II) et d'un métal alcalin, d'un métal alcalino-terreux, ou d'ammonium, comprenant les sels obtenus avec des bases organique d'ammonium, ou des sels d'un composé de formule générale (II) et d'acide organique ou inorganique.

Des sels convenant plus particulièrement à l'invention peuvent être des sels de sodium, de potassium, de calcium, de magnésium, des sels d'ammonium quaternaire tels que tétraméthylammonium ou le tétraéthylammonium, and des sels d'addition avec l'ammoniac et des amines organiques pharmaceutiquement acceptables, tel que la méthylamine, la diméthylamine, la triméthylamine, l'éthylamine, la triéthylamine, l'éthanolamine or la tris-(2-hydroxyéthyl)-amine.

Des sels d'un composé de formule générale (II) et d'acide inorganique convenant à l'invention peuvent être obtenus avec l'acide hydrochlorique, l'acide hydrobromique, l'acide sulfurique, l'acide nitrique ou l'acide phosphorique.

Des sels d'un composé de formule générale (II) et d'acide organique convenant à l'invention peuvent être obtenus avec ou des acide carboxyliques et des acides sulfoniques tel que l'acide formique, l'acide acétique, l'acide oxalique, l'acide citrique, l'acide lactique, l'acide malique, l'acide succinique, l'acide malonique, l'acide benzoïque, l'acide maléique, l'acide fumarique, l'acide tartarique, l'acide méthanesulfonique, l'acide benzènesulfonique ou l'acide p-toluènesulfonique.

Les prodrogues selon l'invention conviennent particulièrement pour le traitement et/ou la prévention de maladies ou de troubles impliquant la surexpression ou la libération d'enzymes intracellulaires qui peuvent cliver le groupe labile E.

A titre de maladies susceptibles d'être considérées par l'invention, on peut notamment citer les cancers, les maladies auto-immunes ou inflammatoires, les maladies immunologiques ou métaboliques, ou les arthropathies.

De manière préférée, les prodrogues de l'invention conviennent en particulier au traitement et/ou la prévention des cancers, les maladies inflammatoires, et les arthropathies.

De manière plus préférée encore, les prodrogues de l'invention conviennent en particulier au traitement et/ou la prévention d'un cancer choisi parmi les cancers du poumon, les cancers du sein, les cancers du colon, les cancers du rein, les tumeurs cérébrales, et les leucémies.

Selon un mode de réalisation, l'invention concerne une composition pharmaceutique ou diagnostique comprenant au moins une quantité efficace d'au moins un composé de formule générale (II) tel que défini précédemment, ou un de ses sels pharmaceutiquement acceptables.

Une composition selon l'invention peut comprendre au moins un excipient pharmaceutiquement acceptable, tels que des additifs, des liants, des agents de gonflement ou lubrifiants, ou des agents de solubilisation habituellement mis en oeuvre dans le domaine.

A titre d'excipient pharmaceutiquement acceptable convenant à l'invention, on peut citer le carbonate de magnésium, le dioxyde de titane, le lactose, le mannitol et d'autres sucres, le talc, les lactoprotéines, la gélatine, l'amidon, la cellulose et ses dérivés, les huiles animales et végétales, comme l'huile de foie de morue, de tournesol, de noix ou de sésame, le polyéthylène glycol, et des solvants tels que l'eau et des mono-ou polyalcools tels que le glycérol.

Le cas échéant, une composition de l'invention peut comprendre d'autres composés actifs appropriés.

Une composition selon l'invention peut être formulée sous toute forme galénique appropriée telle qu'une solution injectable, des comprimés, des gélules, ou des patchs transdermiques.

Le choix de la forme galénique et des excipients pharmaceutiquement acceptables à mettre en oeuvre, ainsi que le cas échéant d'autres composés actifs, dépendent, notamment de la voie d'administration considérée et de la pathologie à traiter ou prévenir, et relèvent des connaissances générales et de la pratique habituelle de l'homme de l'art.

Au sens de l'invention on entend par « quantité efficace », la quantité nécessaire et suffisante pour obtenir l'effet recherché selon l'invention, à savoir l'activité pharmacologique, thérapeutique ou diagnostique, du composé actif D après administration d'une prodrogue selon l'invention. Selon un mode de réalisation préféré, un effet pharmacologique recherché peut être un effet de prévention et/ou de traitement à l'égard d'un cancer.

Une quantité efficace dépend de la nature du composé actif D, de l'effet pharmacologique recherché, de l'individu à qui est administrée une prodrogue de l'invention et de sa condition physiopathologique.

Une quantité efficace peut aisément être déterminée par toute méthode connue de l'homme de métier, notamment, par exemple, au travers d'essais cliniques.

Au sens de l'invention, on entend par «prévention» ou «prévenir» à l'égard d'une maladie, et notamment d'un cancer, le fait de réduire le risque de survenue de cette maladie.

Selon un mode de réalisation, une composition pharmaceutique de l'invention peut comprendre un composé de formule générale (II) ou un de ses sels pharmaceutiquement acceptable, dans laquelle le composé actif D représente un actif anti-cancéreux, en particulier tel que défini ci-après, et la composition est destinée à la prévention et/ou au traitement d'un cancer.

### Composé actif D

Un composé actif D convenant à l'invention peut être choisi parmi les composés à activité thérapeutique et/ou à activité diagnostique.

Plus particulièrement, un composé actif D selon l'invention peut être choisi parmi des composés thérapeutiques actifs vis-à-vis de maladies du tractus gastro-intestinal, de maladies du système cardiovasculaire, de maladies du système nerveux central, de la douleur, de maladies de l'humeur, de maladies du système ophtalmique, de maladies du tractus oropharyngé, de maladies du tractus respiratoire, de maladies des sécrétions endocrines et du métabolisme, de maladies du système reproducteur et urinaire, d'infections virales, bactériennes ou parasitaires, de maladies du système immunitaire, de maladies allergiques, de cancers, ou actifs en obstétrique et en gynécologie, de la peau, ou choisis parmi des composés à visées contraceptives.

Selon un mode de réalisation un composé actif D selon l'invention peut être choisi parmi des hormones, des agonistes ou des antagonistes d'une hormone, telle que la progésterone, la buséreline, le tamoxiféne, la mifépristone ou l'onapristone, parmi des anti-inflammatoires, tels que des anti-inflammatoires non-stéroïdiens, des composés stéroïdiens, des analgésiques, des antipyrétiques, des anesthésiques locaux, des anti-arythmiques, tels que les antagonistes des canaux calciques, des anti-histaminiques, des sympathomimétiques, des inhibiteurs de l'urokinase, des antidépresseurs, des antihypertenseurs, des actifs anticancéreux, des composés réduisant la résistance cellulaire aux cytostatiques, tels que les inhibiteurs de la calmoduline, les inhibiteurs de protéine kinase C, les inhibiteurs de glycoprotéine-P, les modulateurs de l'hexokinase liée au mitochondries, les inhibiteurs de la g-glutamylcystéine synthétase ou de la glutathion-S-transférase, les inhibiteurs de la superoxide dismutase, ou parmi des composés immunosuppresseurs, tels que les macrolides, la cyclosporine A, la rapamycine, le FK 506, l'azothioprine, le méthotrexate, la cyclophosphamide ou le chlorambucile.

Encore plus particulièrement, un composé actif D selon l'invention peut être un actif anticancéreux choisi parmi des cytostatiques, des antimétabolites, des substances intercalantes de l'ADN, des composés inhibiteurs des topoisomérases I and II, des inhibiteurs de tubuline, des agents alkylants, la néocarcinostatine, la calichéamicine, la dynémicine or l'espéramicine A, des composés inhibiteurs des ribosomes, des inhibiteurs des tyrosine phosphokinases, des composés induisant la différentiation cellulaire, ou des inhibiteurs de l'histone déacétylase.

Encore plus particulièrement, un composé actif D selon l'invention peut être un actif anticancéreux choisi parmi des cytostatiques et des antimétabolites, telle que la 5-fluorouracile, la 5-fluorocytidine, la 5-fluorouridine, la cytosine arabinoside ou le méthotrexate, parmi des substances intercalantes de l'ADN, telle que la doxorubicine, la daunomycin, l'idarubicin, l'épirubicin ou le mitoxantrone, parmi des composes inhibiteurs des topoisomérases I and II, telles que la camptothécine, l'étoposide ou le m-AMSA, parmi des inhibiteurs de tubuline, telle que la vincristine, la vinblastine, la vindésine, le taxol, le nocodazole ou la colchicine, parmi des agents alkylants, telle que la cyclophosphamide, la mitomycin C, la rachelmycine, le cisplatine, le gaz moutarde phosphoramide, le melphalan, la bléomycine, la N-bis(2-chloroethyl)-4-hydroxyaniline, ou parmi la néocarcinostatine, la calichéamicine, la dynémicine or l'espéramicine A, ou parmi des composés inhibiteurs des ribosomes, telle que la verrucarine A, parmi des inhibiteurs des tyrosine phosphokinases, telle que la quercétine, la génistéine, l'erbstatine, la tyrphostine ou la rohitukin et ses dérivés, parmi des composés induisant la différentiation cellulaire, tel que l'acide rétinoïque, l'aide butyrique, les esters de phorbol ou l'aclacinomycine, parmi des inhibiteurs de l'histone déacétylase, tel que CI-994 ou MS275.

Selon un mode de réalisation particulièrement préféré de l'invention, un composé actif D convenant à l'invention peut être choisi parmi CI-994 et la doxorubicine, et le MS275.

Selon un autre mode de réalisation, un composé actif D convenant à l'invention peut être un composé diagnostique, notamment choisi parmi des radioligands.

Un radioligand convenant à l'invention peut être un composé porteur d'un groupe radioactif, notamment choisi parmi ³H, ¹⁴C, ³⁵S, ¹³¹I, ¹²⁵I ou ¹⁸F.

Selon un mode de réalisation, D peut être constitué d'un composé actif ou alternativement d'une pluralité de composés actifs. Avantageusement, lorsque D est constitué d'une pluralité de composés actifs, ceux-ci sont reliés entre eux par un amplificateur chimique auto-immolable. Lorsque plusieurs composés actifs sont présents, ils peuvent être identiques ou différents.

Un amplificateur convenant à l'invention comprend une pluralité de fonctions réactives, et de préférence, au moins trois fonctions réactives de sorte à permettre sa liaison à un bras réactif de l'invention et à au moins deux composés actifs. Il est en outre doté de la capacité à subir une décomposition spontanée propice à la libération des composés actifs, après sa libération à partir d'un bras réactif de l'invention. De préférence encore un amplificateur auto-immolable convenant à l'invention peut comprendre au moins 3, voire au moins 4 fonctions réactives.

A titre d'exemple d'amplificateur convenant à l'invention, on peut citer le 2,4-bis(hydroxyméthyl)aniline, le 2,4,6-ter(hydroxyméthyl)aniline, le 2,4 bis(hydroxy-méthyl)phénol, ou le 2,4,6-ter(hydroxyméthyl)phénol. Ces amplificateurs auto-immolables sont liés au bras réactif de l'invention par le biais de la fonction phénol ou aniline.

De manière préférée, un amplificateur auto-immolable convenant à l'invention peut-être le 2,4-bis(hydroxyméthyl)aniline.

Selon encore un autre mode de réalisation, un amplificateur convenant à l'invention peut comprendre un enchaînement d'au moins deux, voir 3, ou encore 4 unités amplificatrices auto-immolables tels que définies ci-dessus. Chaque unité amplificatrice auto-immolable d'un tel enchaînement est lié à au moins une autre unité amplificatrice auto-immolable et à au moins un composé actif. Les composés actifs sont libérés en cascade, consécutivement à la décomposition spontanée de chaque unité amplificatrice, également libérée en cascade, suite à la libération de l'unité liée au bras réactif de l'invention.

Un amplificateur de l'invention peut-être de formule générale (III) suivante : 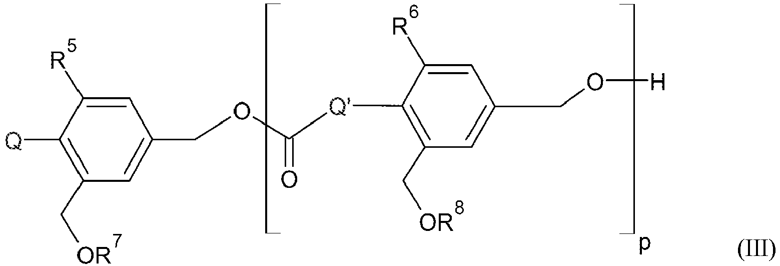 dans laquelle
- p peut représenter un entier naturel variant de 0 à 3, et de préférence de 1 à 2. De préférence encore, p vaut 0, 1 ou 2.
- Q peut représenter -NH₂ ou -OH, et de préférence représente -NH₂,
- chaque Q' peut représenter, indépendamment l'un de l'autre, NH ou O, et de préférence représente NH,
- R⁵ et R⁶ peuvent représenter, indépendamment l'un de l'autre, H ou -CH₂OR⁹, avec R⁹ pouvant représenter R⁷ ou R⁸ tel que défini ci-après,
- R⁷ et R⁸ peuvent représenter, indépendamment l'un de l'autre, H ou une sous-unité de formule générale (IV) suivante : 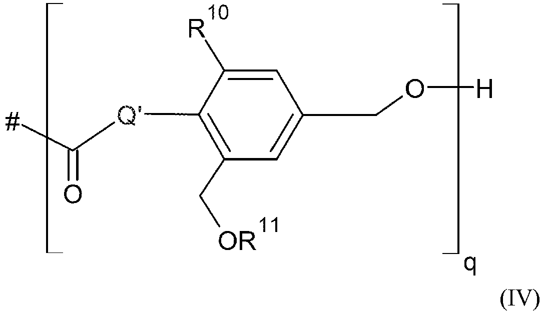 dans laquelle
   - # figure une liaison avec un oxygène benzylique,
   - q peut représenter un entier naturel variant de 0 à 3, et de préférence de 1 à 2. De préférence encore, q vaut 0, 1 ou 2,
   - Q' est tel que défini ci-dessus,
   - R¹⁰ peut représenter R⁵ ou R⁶ tel que défini ci-dessus, et
   - R¹¹ peut représenter R⁷ ou R⁸ tel que défini ci-dessus.

De préférence, au moins un de R⁵ ou de R⁶ peut représenter H et au moins un de R⁵ ou de R⁶ peut représenter -CH₂OR⁹, avec R⁹ étant tel que défini ci-dessus.

De préférence, au moins un de R⁷ ou de R⁸ peut représenter H et au moins un de R⁷ ou de R⁸ peut représenter une sous-unité de formule générale (IV) tel que défini ci-dessus.

Avantageusement, un amplificateur auto-immolable convenant à l'invention défini une structure dendrimèrique avec au moins deux, voire 3, ou 4 générations.

Selon un mode de réalisation, D, dans un composé de formule générale (II) tel que défini précédemment, peut figurer un composé de formule générale (V) : 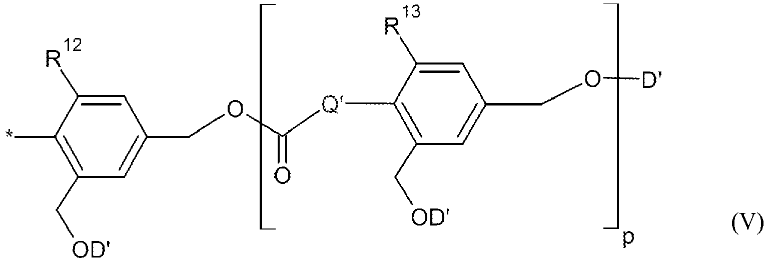 dans laquelle
- Q' et p sont tels que définis ci-dessus,
- * figure une liaison avec A tel que défini à l'égard de la formule générale (II),
- R¹² et R¹³ peuvent représenter, indépendamment l'un de l'autre, H ou -CH₂OD', avec D' étant tel que défini ci-après, et
- D' peut figurer un composé actif tel que défini ci-dessus, ou une sous-unité de formule générale (VI) suivante : dans laquelle 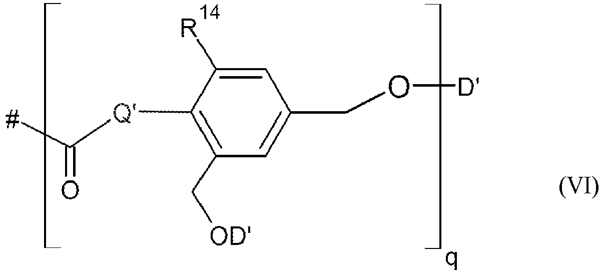
   - #, Q', D', et q sont tels que définis précédemment,
   - R¹⁴ peut représenter, H ou -CH₂OD', avec D' étant tel que défini ci-avant.

Avantageusement, un composé D de l'invention peut présenter une structure dendrimèrique avec au moins deux, voire 3, ou 4 générations.

### Groupe labile E

Selon un mode de réalisation, un groupe labile E convenant à l'invention peut en particulier être un substrat enzymatiquement hydrolysable.

Un groupe labile E convenant à l'invention est tel que son élimination, par exemple par voie enzymatique ou consécutivement à une variation de pH, assure, par réarrangement intra-moléculaire du bras auto-réactif, une libération du composé actif D.

Un groupe labile E selon l'invention, notamment enzymatiquement hydrolysable, peut être plus particulièrement sélectionné pour conférer une spécificité tissulaire et/ou cellulaire aux prodrogues selon l'invention.

Selon un mode de réalisation préféré, un groupe labile E convenant à l'invention peut être enzymatiquement hydrolysable.

Selon un mode de réalisation, un groupe labile E selon l'invention, enzymatiquement hydrolysable, peut être choisi de sorte à être hydrolysé par une ou des enzymes dont l'expression est spécifiquement attachée à un tissu cible ou des cellules cibles.

Parmi les enzymes convenant à l'invention, on peut notamment mentionner les carboxylestérases, les alkaline phosphatases, les glucuronidases, et notamment la β-glucuronidase, les glycosidases, les protéases, les peptidases, ou les métalloprotéases.

La sélection d'un groupe labile E substrat approprié d'une enzyme appartenant au groupe défini ci-dessus relève des compétences générales de l'homme du métier.

Selon un mode préféré de réalisation, un groupe labile E peut être un substrat d'une enzyme choisie parmi les glucuronidases, et notamment la β-glucuronidase, les glycosidases, les protéases, les peptidases, et les métalloprotéases.

Selon une variante préférée de réalisation, un groupe labile E enzymatiquement hydrolysable convenant à l'invention peut être un substrat choisi parmi les substrats de la galactosidase, et en particulier de la β-galactosidase, de l'induronidase, de la glucosidase, de la N-acétyl-D-glucosaminidase, de la N-acétyl-D-galactosaminidase, de la mannosidase, de la fucosidase ou de la glucuronidase, notamment de la β-glucuronidase, les substrats des cathepsines, ou les substrats des métalloprotéases, notamment de la PSMA (Prostate Specific Membrane Antigen).

Selon une variante encore plus préférée de l'invention, un groupe labile E enzymatiquement hydrolysable convenant à l'invention peut être un substrat d'une enzyme choisie parmi la β-glucuronidase, la β-galactosidase, les cathepsines, ou les métalloprotéases, et notamment le PSMA.

Selon un mode de réalisation, un groupe labile E enzymatiquement hydrolysable convenant à l'invention peut notamment être choisi parmi un groupe glycosyle ou un groupe peptidique.

Selon une variante de réalisation, un groupe labile E enzymatiquement hydrolysable convenant à l'invention peut notamment être choisi parmi un groupe glycosyle, notamment un groupe D-glucuronyle, un groupe L-iduronyle, un groupe D-glucopyranosyle, un groupe D-galactopyranosyle, un groupe N-acétyl-D-glucosaminyle, un groupe N-acétyl-D-galactosaminyle, un groupe D-mannopyranosyle, un groupe L-fucopyranosyle.

Selon une autre variante de réalisation, un groupe labile E enzymatiquement hydrolysable convenant à l'invention peut notamment être un polysaccharide comprenant de 2 à 20, notamment de 3 à 10, et plus particulièrement de 4 à 6 unités d'ose choisies parmi les groupes osidiques définis ci-dessus.

Selon une autre variante préférée de réalisation, un groupe labile E enzymatiquement hydrolysable convenant à l'invention peut être un groupe choisi parmi un groupe D-glucuronyle, un groupe D-galactopyranosyle, un groupe D-glucopyranosyle, ou un groupe mannopyranosyle.

De manière plus préférée encore, un groupe labile E enzymatiquement hydrolysable convenant à l'invention peut être un groupe D-glucuronyle.

La β-glucuronidase est une enzyme présente naturellement en concentration importante dans le voisinage de nombreuses tumeurs. Les prodrogues de l'invention comprenant à titre de groupe labile E un groupe glucuronyle peuvent donc être avantageusement activées au niveau extracellulaire, au cours de monothérapies à prodrogue ou PMT (Prodrug Mono-Therapy). Par ailleurs, la β-glucuronidase et la β-galactosidase sont deux enzymes lysosomales qui sont présentes dans la plupart des cellules malignes. Ainsi, l'activation des prodrogues glucuronylées ou galactosylées par les enzymes correspondantes peut être réalisée dans le milieu intracellulaire après leur internalisation par endocytose.

Selon un autre mode de réalisation, un groupe labile E enzymatiquement hydrolysable peut être un substrat d'une protéase ou d'une peptidase, notamment un substrat d'une cathepsine ou d'une métalloprotéase, en particulier de la PSMA.

Selon un autre mode de réalisation, la spécificité tissulaire et/ou cellulaire par un groupe labile E enzymatiquement hydrolysable peut être assurée après adressage de l'enzyme apte à hydrolyser le groupe E vers les tissus ou cellules ciblés, par exemple au moyen d'un anticorps.

Dans ce mode de réalisation, l'enzyme est préalablement fixée à un anticorps spécifique des tissus ou cellules ciblé(e)s, et le conjugué ainsi obtenu est administré préalablement à l'administration d'une prodrogue de l'invention.

Ce type de procédé est connu de l'homme de l'art, et est dénommé thérapie à prodrogue dirigée par enzyme ou ADEPT (Antibody Directed Enzyme Prodrug Therapy).

Il relève des compétences de l'homme de l'art de sélectionner la nature du groupe X d'un bras auto-réactif selon l'invention figuré par la formule générale (I) ou (Ia) en fonction de la réaction enzymatique, et donc de l'enzyme, qui doit conduire à l'élimination du groupe labile E.

Dans le cas des estérases, X sera choisi pour que E et X' forment ensemble une fonction ester ou carbonate.

Dans le cas des glycosidases, X sera choisi pour que E et X' forment ensemble une liaison glycosidique.

Dans le cas des peptidases et des protéases, X sera choisi pour que E et X' forment ensemble une fonction amide.

Selon un mode de réalisation lorsque que le groupe labile E est un groupe glycosyle, par exemple un groupe D-galactopyranosyle ou un groupe D-glucuronyle, X peut être de préférence un groupe OH.

Ainsi, selon un mode de réalisation préféré, un composé de formule générale (II) convenant à l'invention peut être tel que X' représente O et E représente un groupe glycosyle, de préférence choisi parmi un groupe D-galactopyranosyle, un groupe D-glucuronyle, un groupe D-glucopyranosyle, ou un groupe mannopyranosyle.

Selon un autre mode de réalisation lorsque que le groupe labile E est de nature peptidique ou protéique, X peut être de préférence un groupe NH₂.

Ainsi, selon un mode de réalisation, un composé de formule générale (II) convenant à l'invention peut être tel que X' représente NH et E représente un groupe peptidique substrat des cathepsines ou des protéases, et en particulier substrat des cathepsines tel que Ala-Leu-Ala-Leu.

Selon un autre mode de réalisation préféré E peut représenter un groupe peptidique substrat de la PSMA.

### Ligand de ciblage L

Un ligand de ciblage convenant à l'invention permet avantageusement d'adresser une prodrogue de l'invention vers un organe, un tissu ou des cellules spécifiques.

Plus particulièrement, un ligand de ciblage L convenant à l'invention permet avantageusement d'adresser une prodrogue de l'invention vers une tumeur ou des cellules malignes.

Selon un mode de réalisation, un ligand de ciblage L peut en outre comprendre un groupe Z destiné à moduler la solubilité, et en particulier améliorer la solubilité dans les fluides biologiques, d'une prodrogue de l'invention.

Selon un mode de réalisation, un ligand de ciblage convenant à l'invention peut être de la forme L-(Z)ₙ- dans laquelle n peut être égale à 0 ou 1, et L et Z sont notamment tels que définis ci-après.

Un ligand de ciblage L peut être choisi parmi un peptide, une protéine, un anticorps ou un fragment d'anticorps reconnaissant un antigène, un ligand d'un récepteur cellulaire, un biopolymère, un monosaccharide, un oligosaccharide, une hormone, une vitamine, un dendrimère, une polyamine, ou une nanoparticule, métallique ou non.

Un ligand de ciblage L convenant à l'invention peut être choisi parmi les ligands aptes à cibler, par exemple, les intégrines, la nucléoline, l'aminopeptidase N, l'endogline, le récepteur du facteur de croissance endothélial vasculaire, les récepteurs des lipoprotéines de faible densité (LDL), le récepteur de la transferrine, les récepteurs de la somatostatine, de la bombésine, du Neuropeptide Y, le récepteur de l'hormone de libération de l'hormone lutéinisante, les récepteurs de l'acide folique, les récepteurs des facteurs de croissance de l'épiderme (EGF), les récepteurs du facteur de croissance transformant (TGF), les récepteurs du facteur de croissance des fibroblastes (FGF), les récepteurs des asialoglycoprotéines, les récepteurs des galectines, les récepteurs des sélectines, ou les récepteurs de l'acide hyaluronique.

Un ligand de ciblage L convenant à l'invention, peut notamment être un ligand décrit par Krat et al. (Chem. Med. Chem., 2008,3:20).

On entend par "biopolymère", un polymère biocompatible. Un biopolymère convenant à l'invention peut être le copolymère *N*-(2-hydroxypropyl)méthacrylamide. Un tel polymère peut assurer le ciblage d'une prodrogue de l'invention vers les tumeurs par le phénomène de rétention et de perméabilité augmenté (EPR, enhanced permeability and rétention effect) lié à la vascularisation spécifique des tumeurs.

Un monosaccharide convenant à l'invention peut par exemple être le glucose, le galactose, ou le mannose.

Un oligosaccharide convenant à l'invention peut par exemple être le lactose.

Un peptide convenant à l'invention peut par exemple être un peptide RGD, des peptides se liant aux parois des artères, un peptide issu de EGF, ou un peptide issu du NGF

Une protéine convenant à l'invention peut par exemple être une lectine, telle que la concanaline A ou la mannan-binding lectin, une sélectine, une galectine, un facteur de croissance, tel que le VEGF, le FGF ou le NGF, les protéines du surfactant A et B, des asialoglycoprotéines, des protéines du circumsporozoite du paludisme, ou la protéine Rap.

Un anticorps ou un fragment d'anticorps reconnaissant un antigène convenant à l'invention peut par exemple être un anticorps anti-CD3, un anticorps anti-CD5, un anticorps anti-CD117, un anticorps anti-ErbB2, une IgG, un anticorps anti-HER2, un anticorps ChCE7, un anticorps OV-TL16 Fab', un anticorps anti-PECAM, un anticorps anti-thrombomoduline, ou un anticorps anti-Tn, un anticorps anti-CD20, un anticorps anti-CD33, un anticorps anti-CEA, un anticorps anti-glycoprotéines du type mucine (MUC-1, CanAg, Lewis Y, Lewis X), un anticorps anti-CT7, un anticorps anti-MAGE, ou un anticorps anti-PSMA.

Un ligand d'un récepteur cellulaire convenant à l'invention peut par exemple être l'acide folique, le Neuropeptide Y, la leptine, un ligand du récepteur de l'EGF, du FGF, du TGF ou du NGF, l'insuline, un ligand synthétique mannosylé, la transferrine ou une catécholamine.

Une hormone convenant à l'invention peut par exemple être la progestérone, un oestrogène, la testostérone, l'hormone anti-diurétique, une hormone de croissance, ou une hormone thyroïdienne.

Une polyamine convenant à l'invention peut par exemple être une polylysine.

Une nanoparticule convenant à l'invention peut par exemple être une nanoparticule métallique ou une nanoparticule non métallique.

Selon un mode de réalisation préféré, un ligand de ciblage L peut représenter un ligand d'un récepteur cellulaire, et de préférence est l'acide folique.

En effet, le récepteur de l'acide folique est surexprimé dans de nombreuses tumeurs telles que les tumeurs des ovaires, des poumons, du sein, du rein, du cerveau, de l'endomètre, du colon, ou dans les leucémies. En revanche, celui-ci est peu, ou pas, présent à la surface des cellules saines.

Selon un mode de réalisation, un ligand de ciblage convenant à l'invention peut être de forme L-(Z)ₙ- avec n = 0 ou 1. Z peut être en particulier destiné à moduler la solubilité, et notamment à améliorer la solubilité dans les fluides biologiques, d'une prodrogue de l'invention.

Le choix de la nature du groupe Z est déterminé, d'une part, en fonction de la nature du composé actif D, du groupe labile E et du ligand de ciblage L fixé sur un bras auto-réactif de l'invention, et, d'autre part, en fonction des paramètres de solubilité de la prodrogue à ajuster.

Selon un mode de réalisation, n peut être égale à 0 ou 1, et Z peut représenter un groupe alkylène, linéaire ou ramifié, saturé ou insaturé, en C₁-C₁₀, optionnellement interrompu par un ou plusieurs hétéroatomes choisis parmi O ou N, un groupe glycosyle, un groupe O-(CHR³-CHR⁴-O-)ₘ ou N-(CHR³-CHR⁴-O-)ₘ dans lesquels m figure un entier naturel variant de 1 à 20, R³ et R⁴ figurent, indépendamment l'un de l'autre, H ou CH₃, sous réserve que R³ et R⁴ ne figurent pas simultanément CH₃, un groupe issu d'un acide aminé ou d'un peptide, ou une combinaison de ces groupes

Selon un mode de réalisation, n peut être égale à 1, et Z peut représenter un groupe alkylène en C₁-C₅, de préférence en C₁-C₃, et plus préférentiellement un méthylène.

Selon un autre mode de réalisation, n peut être égale à 1, et Z peut représenter un groupe glycosyle choisi parmi un groupe glucosyle, un groupe galactosyle, un groupe mannosyle, un groupe lactosyle.

Selon une variante de réalisation, Z peut représenter un groupe glycosyle tel que défini précédemment.

Selon un autre mode de réalisation, n peut être égale à 1, et Z peut représenter un groupe O-(CHR³-CHR⁴-O-)ₘ ou N-(CHR₃-CHR₄-O-)ₘ dans lesquels m figure un entier naturel variant de 2 à 18, de préférence de 3 à 16, et plus préférentiellement de 4 à 12.

Selon un autre mode de réalisation, n peut être égale à 1, et Z peut représenter un groupe issu d'un acide aminé ou d'un peptide. A titre d'exemple d'acides aminés convenant à l'invention on peut en particulier citer la tyrosine.

Selon un autre mode de réalisation, n peut être égale à 1, et Z peut représenter une combinaison des groupes précédemment définis. En particulier, Z peut représenter une combinaison d'un groupe glycosyle et d'un groupe O-(CHR³-CHR⁴-O-)ₘ ou N-(CHR₃-CHR₄-O-)ₘ tels que définis ci-dessus.

Selon un autre préféré de réalisation, n peut être égale à 0.

### Préparation d'une prodrogue

Un composé de formulé générale (II) peut être préparé à partir d'un composé de formule générale (I) et des groupes D-v, E-w, et L-(Z)ₙ-x, notamment tels que définis ci-après par tout procédé connu de l'homme de l'art.

En particulier un composé de formulé générale (II) peut être obtenu par un procédé de préparation consistant à faire réagir un composé de formule générale (I) ou (Ia) tel que défini précédemment avec des groupes D-v, E-w, et L-(Z)ₙ-x, dans lesquels D, E et L et Z sont tels que définis précédemment, et v, w et x figurent chacun une fonction réactive telle que, à l'égard du composé de formule générale (I), v réagit avec OH lié au carbone benzylique, ou ledit OH préalablement activé, w réagit X, et x réagit avec F.

Les fonctions réactives v, w et x peuvent être naturellement présentent sur les groupes D, E et L-(Z)ₙ, ou peuvent être, préalablement à l'étape de réaction, introduites sur les groupes D, E et L-(Z)ₙ.

Il relève des compétences de l'homme de l'art de modifier les groupes D, E et L-(Z)ₙ afin d'y introduire les fonctions réactives v, w et x appropriées à l'invention.

En particulier, v, w et x sont tels que définis ci-après.

Un procédé de préparation d'une prodrogue selon l'invention peut comprendre au moins les étapes consistant à faire réagir :
a- un composé de formule générale (I) ou (Ia) avec un composé E-w,
b- le produit de la réaction de l'étape a- avec un composé D-v, et
c- le produit de la réaction de l'étape b- avec un composé L-(Z)ₙ-,
dans lequel les composé de formule générale (I) ou (Ia), E-w, D-v, et L-(Z)ₙ- sont notamment tels que définis ci-après.

Un procédé de l'invention peut en outre comprendre à l'issue de chaque étape a-, b- et c- une étape de purification du produit obtenu.

Plus particulièrement, les étapes a-, b- et c- d'un procédé de l'invention peuvent, notamment, être telles que définies ci-après.

Selon un mode de réalisation, un bras auto-réactif selon l'invention peut être obtenu par un procédé comprenant au moins une étape consistant à faire réagir chimiquement un composé de l'invention figuré par la formule générale (I) avec un groupe D-v, dans lequel D est tel que défini précédemment et v représente une fonction réactive telle que, à l'égard du composé de formule générale (I), v réagit avec OH lié au carbone benzylique.

Selon une variante de réalisation, la fixation par voie chimique d'un composé actif D à la fonction OH du carbone benzylique d'un composé de l'invention de formule générale (I) peut comprendre une étape préalable d'activation de la fonction OH, suivie d'une étape de réaction d'un groupe D-v avec la fonction OH activée.

Selon un mode de réalisation, la fonction OH lié au carbone benzylique d'un composé de l'invention de formule générale (I) peut être activée par réaction dudit composé avec un composé choisi parmi le phényle chloroformiate et ses dérivés tel que le para-nitrophényle chloroformiate, le dinitrophényle chloroformiate, le fluorophényle chloroformiate, ou le carbonyle de diimidazole.

Selon un mode de réalisation, v est choisi de telle sorte que la réaction chimique d'un groupe D-v avec la fonction OH liée au carbone benzylique d'un composé de formule générale (I), ou ladite fonction OH préalablement activée, conduit à la formation d'un groupe - A(CO)O- liant le groupe D au carbone benzylique, dans lequel A peut figurer O, et ledit groupe représente une fonction carbonate, ou A peut figurer NH, et ledit groupe représente une fonction carbamate.

Selon un mode de réalisation préféré, un bras réactif de l'invention est mis en oeuvre avec une fonction OH liée au carbone benzylique préalablement activé, comme indiquée ci-dessus.

Selon encore un autre mode de réalisation préféré, un groupe D-v est mis à réagir avec une fonction OH liée au carbone benzylique activée par réaction avec un phényle chloroformiate et un de ses dérivés, comme indiqué ci-dessus.

De manière préférée, v est choisi de telle sorte que dans le groupe -A(CO)O-, A figure NH.

Il relève des compétences de l'homme de l'art de sélectionner la nature du groupe v selon que la réaction chimique sera effectuée avec la fonction OH ou une fonction OH activée du carbone benzylique d'un composé de formule générale (I).

Lorsque D figure une pluralité de composés actifs liés à un amplificateur comme défini ci-dessus, v dans la formule D-v figure la fonction aniline ou phénol de l'amplificateur.

Selon un mode de réalisation, v peut être choisi parmi NH₂, NHR", OH, ou SH, avec R" étant tel que défini précédemment. Une fonction réactive v peut être présente dans un groupe fonctionnel, par exemple -CONHOH, -CONR"OH, -CONH₂, -CONHR" ou -NHOH, avec R" étant tel que défini précédemment.

Selon un autre mode préféré de réalisation, les composés actifs D peuvent être couplés à l'alcool benzylique du bras auto-réactif de l'invention préalablement activé sous forme d'un carbonate de phényle et de ses dérivés tels qu'un carbonate de *p*-nitrophényle, un carbonate de dinitrophényle, un carbonate de fluorophényle, ou d'un carbonate de diimidazole.

Selon un mode de réalisation, un bras auto-réactif selon l'invention peut être obtenu par un procédé comprenant au moins une étape consistant à faire réagir chimiquement un composé de l'invention figuré par la formule générale (I) avec un groupe E-w, dans lequel E est tel que défini précédemment et w représente une fonction réactive telle que, à l'égard du composé de formule générale (I), w réagit avec X.

Selon un mode de réalisation, lorsque X figure OH, w peut représenter un radical halogéné, notamment Cl ou Br, et en particulier Br.

Selon un autre mode de réalisation, lorsque X figure NH₂, w peut représenter un radical -COOH ou -OC(O)Cl.

Selon un mode particulier de réalisation, les groupes labiles E enzymatiquement hydrolysables de type groupe glycosyle peuvent être introduits sur une fonction phénol d'un bras auto-réactif de l'invention *via* une réaction de glycosylation chimiosélective.

Selon un mode de réalisation, un bras auto-réactif selon l'invention peut être obtenu par un procédé comprenant au moins une étape consistant à faire réagir chimiquement un composé de l'invention figuré par la formule générale (I) avec un groupe L-(Z)ₙ-x, dans lequel L, Z et n sont tels que définis précédemment et x représente une fonction réactive telle que, à l'égard du composé de formule générale (I), x réagit avec F par une réaction de chimie click.

La réaction par chimie click entre les fonctions réactives F et x donne le groupe G de la formule générale (II) définie ci-dessus. La nature du groupe G dépend naturellement de la nature des fonctions réactives de départ F et x.

Les réactions de chimique click et les couples de fonctions réactives F et x sont connues de l'homme de métier. A cet égard on peut se référer à la revue de Kolb et al. (Angew. Chem. Int. Ed., 2001, 40: 2004).

Selon un mode de réalisation, F représente une fonction réactive activable par chimie click choisie parmi -C≡CR"', -N₃, -SH, -C=CH₂, cyclooctynes, maléimide, -SO₂N₃, ou -COSR'" avec R'" représentant H ou un radical alkyle, linéaire ou ramifié, saturé ou insaturé, en C₁ à C₁₀, et notamment en C₂ à C₆, voire en C₃ ou C₄.

Il relève des compétences de l'homme de l'art de sélectionner la nature du groupe x selon la nature d'un groupe F d'un composé de formule générale (I).

Selon un mode de réalisation x et F peuvent être choisis parmi les couples de fonctions réactives activables en chimie click suivants, (-N₃, -C≡CR"'), (-SH, -C=CH₂), (-N₃, cyclooctynes), (-SH, maléimide), (-SO₂N₃, -COSR"'), avec R'" représentant H ou un radical alkyle, linéaire ou ramifié, saturé ou insaturé, en C₁ à C₁₀, et notamment en C₂ à C₆, voire en C₃ ou C₄.

Selon un autre mode particulier de réalisation, un ligand de ciblage L-(Z)ₙ-x, peut être fixé sur l'ensemble groupe E/bras auto-réactif/composé actif par une réaction de «chimie click » ou « click chemistry » avec une fonction alcyne.

Dans l'ensemble de la demande, à savoir la description présentée ci-avant et les exemples présentés ci-après, l'expression « entre... et... » relative à une plage de valeur doit être entendue comme incluant les bornes de cette plage.

Les exemples donnés ci-après sont présentés à titre d'illustration de l'objet de l'invention.

### FIGURES

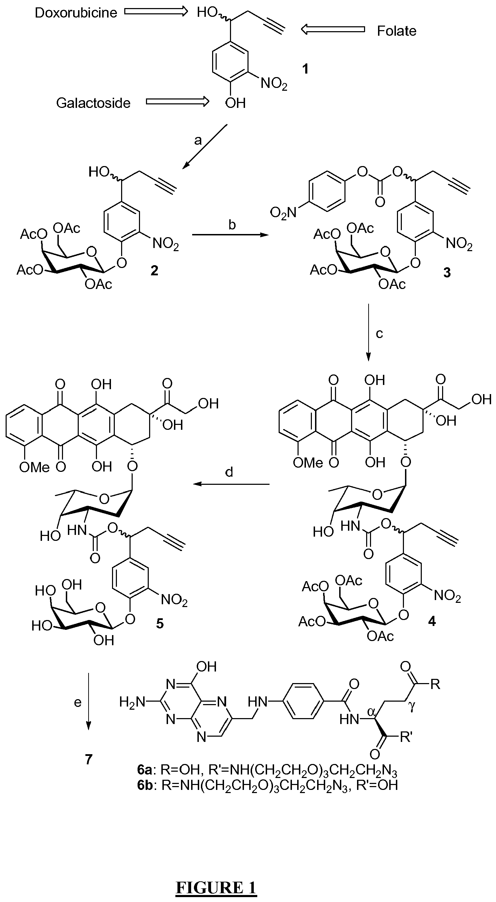

**Figure 1** **:** illustre le schéma de synthèse d'un vecteur selon l'invention, composé 7, par réaction sur un bras auto-réactif de l'invention d'une drogue D, doxorubicine, d'un groupe labile ou gâchette E, galactoside, et d'un ligand de ciblage L, folate. Sur la figure 1, a) représente 1-bromo-(2,3,4,6-*O*-tetra-*O*-acetyl)-β-D-galactopyranoside, Ag₂CO₃, HMTTA, CH₃CN, 84% ; b) représente *p*-nitrophenyl-chloroformate, pyridine, CH₂Cl₂, 92%; c) représente doxorubicin, Et₃N, DMF, 65%; d) représente NaOMe, MeOH/CH₂Cl₂, -15°C, 80%; e) représente CuSO₄, sodium ascorbate, DMSO/H₂O, 75%.

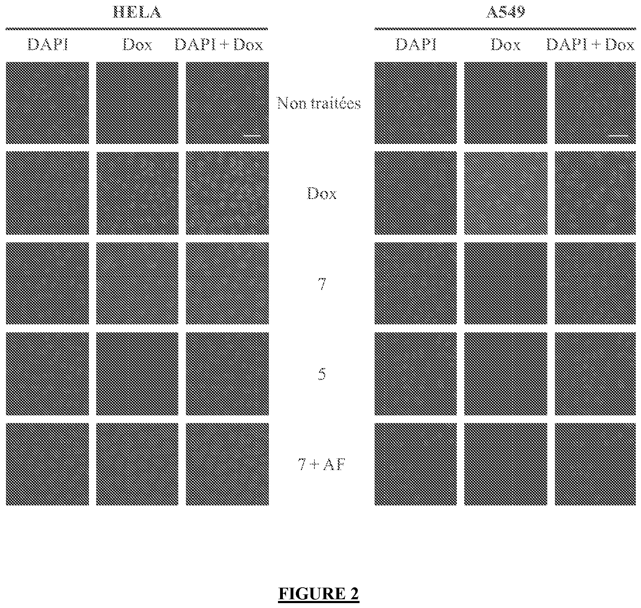

**Figure 2****:** illustre la capacité d'une prodrogue de l'invention (composé **7,** DOX-GAL-AF) à être spécifiquement internalisée dans des cellules exprimant un récepteur pour le ligand de ciblage (HeLa, récepteur à l'acide folique) en comparaison d'un composé non conforme à l'invention (composé **5,** DOX-GAL) et du composé actif non vectorisé sous la forme d'une prodrogue (DOX), et par rapport à des cellules n'exprimant pas un tel récepteur (A549). La saturation du milieu de culture des cellules en acide folique (+AF) prévient l'entrée du composé DOX-GAL-AF dans les cellules HeLa.

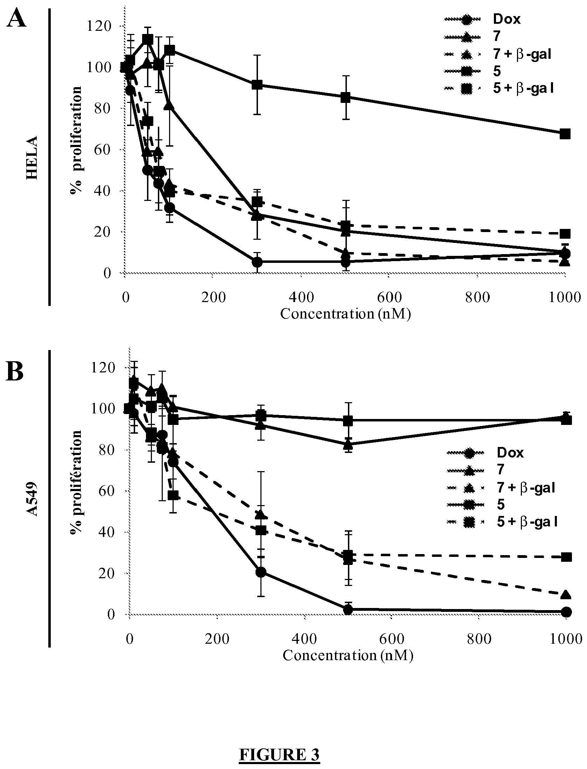

**Figure 3** : illustre la cytotoxicité spécifique d'une prodrogue de l'invention (composé 7, DOX-GAL-AF) dans un milieu de culture dénué (triangle noir, trait continu) ou saturé en β-galactosidase (triangle noir, pointillé), en comparaison d'un composé non conforme à l'invention (composé **5,** DOX-GAL) dans un milieu de culture dénué (carré noir, trait continu) ou saturé en β-galactosidase (carré noir, pointillé), du composé actif non vectorisé sous la forme d'une prodrogue (DOX, rond noir), vis-à-vis de cellules exprimant un récepteur pour le ligand de ciblage (HeLa, récepteur à l'acide folique, Figure 3A) et de cellules n'exprimant pas un tel récepteur (A549, Figure 3B).

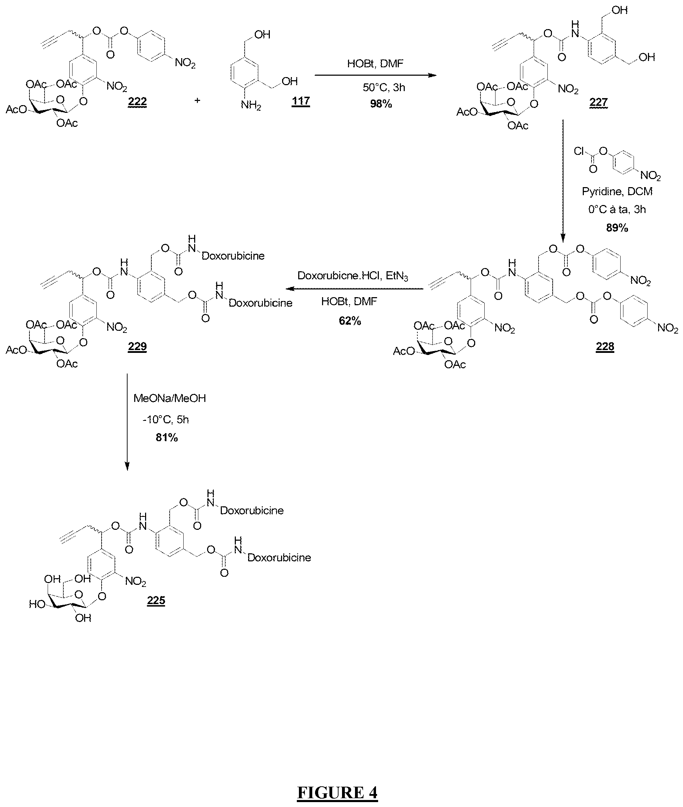

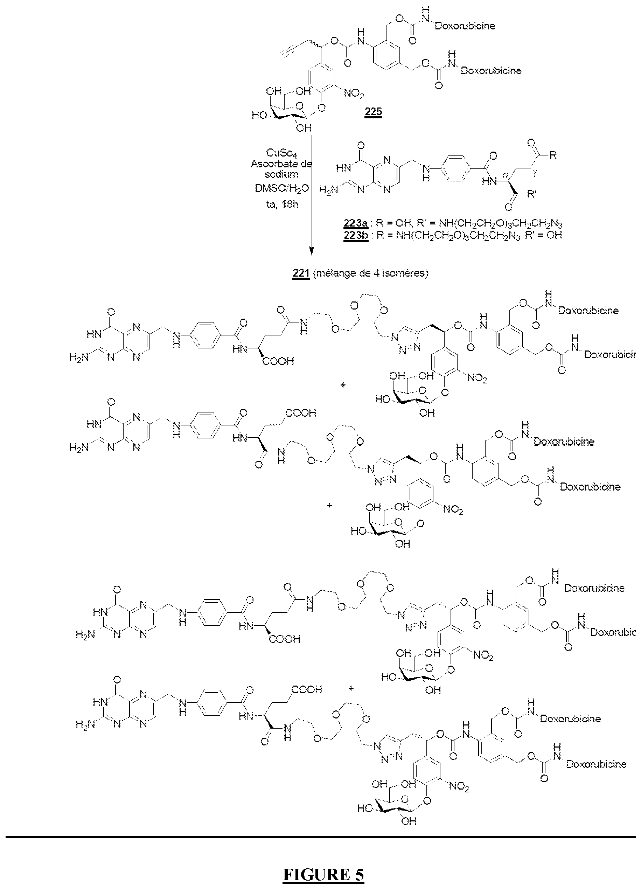

**Figures 4** **et** **5** : illustre le schéma de synthèse d'un vecteur dendritique selon l'invention, composé **(221),** par réaction sur un bras auto-réactif de l'invention d'une drogue D constituée d'une pluralité de composés actifs, doxorubicine, fixés sur un amplificateur, d'un groupe labile ou gâchette E, galactoside, et d'un ligand de ciblage L, folate.

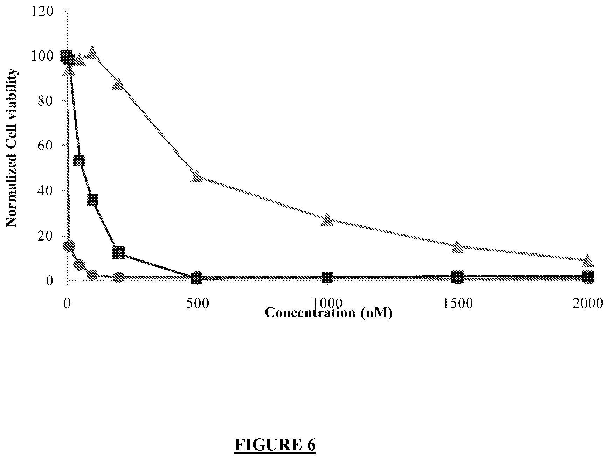

**Figure 6** **:** illustre la cytotoxicité spécifique deux prodrogues de l'invention, composé (7), DOX-GAL-AF, (triangle) et vecteur dendritique **(221)** (carré) par rapport à la Doxorubicine (rond) sur une lignée de cellules cancéreuses LAM de type KG1.

### EXEMPLES

### Exemple 1

### 1- Procédé général de synthèse

Toutes les réactions ont été effectuées sous atmosphère d'azote N₂. Sauf indications contraires, les solvants utilisés sont de qualité Chromatographie Liquide Haute Performance (CLHP).

Les réactifs chimiques mis en oeuvre sont de qualité analytique, obtenus à partir de sources commerciales, et utilisés sans purification additionnelle.

L'évolution des réactions a été suivie en chromatographie sur couche mince (CCM) en utilisant des plaques pré-recouvertes d'un gel de silice MACHEREY-NAGEL ALUGRAM® SILG/UV₂₅₄ (0,2 mm de gel de silice 60). Les taches ont été visualisées après exposition de la plaque en lumière ultra-violette (254 nm) et/ou par trempage de la plaque CCM dans une solution à 3 g d'acide phosphomolibdique dans 100 ml d'éthanol suivi d'un séchage au sèche-cheveux.

La chromatographie flash sur colonne a été réalisée en utilisant un gel de silice MACHEREY-NAGEL 60 (15-40 µm) comme phase stationnaire.

Les spectres RMN ¹H et ¹³C et ont été enregistrés à 400 MHz sur un instrument BRUKER 400 AVANCE III. Les déplacements chimiques (δ) sont rapportés en partie par million en prenant le tétraméthylsilane comme référence interne. Les constantes de couplage (J) sont rapportées en Hertz (Hz).

Les abréviations standard indiquant une multiplicité sont indiquées comme suit : b = large, s = singulet, d = doublet, t = triplet, m = multiplet.

Les points de fusion ont été mesurés sur un appareil Büchi Melting Point b-545 et non corrigés.

La spectrométrie de masse ESI a été réalisée par le Centre Régional des Mesures Physiques de l'Ouest (CRNPO) à l'Université de Rennes 1.

La CLHP analytique en phase inverse (RP-HPLC) a été réalisée sur un dispositif Dionex Ultimate 3000 doté d'un détecteur de longueur d'onde variable UV/Visible et d'une colonne de chromatographie à phase inverse ACCLAIM® (120, C18, 251X4,6 mm, 5 µm, 120 Å) à 30°C et 1 ml.min⁻¹. Le gradient d'élution était composé de A (0,2 % de TFA dans de l'eau) et B (CH₃CN).

L'analyse en spectrométrie de masse couplée à une chromatographie liquide (SM-CL ou LCMS) a été réalisée avec un appareillage WATERS doté d'un détecteur de masse 3100, un module Separator WATERS 2695 et un détecteur de longueur d'onde variable UV/visible WATERS 2489. La colonne pour chromatographie en phase inverse utilisée était une colonne ACCLAIM® (120, C18, 251x4,6 mm, 5 µm, 120 Å à 30 °C et 1 ml.min⁻¹. Le gradient d'élution était composé de A (0,2 % de TFA dans de l'eau) et B (CH₃CN).

La CLHP en phase inverse préparative a été réalisée avec un dispositif VARIAN PREPSTAR. Un débit de solvant de 20 ml.min⁻¹ a été appliqué sur une colonne semi-préparative VARIAN Dynamax (250x21,4 mm Microsorb 100-5 C18). Le gradient d'élution était composé de A (0,2 % de TFA dans de l'eau) et B (CH₃CN).

Méthode 1 (pour l'analyse CLHP et SM-CL) : gradient linéaire commençant avec A/B = 80/20 V/V, atteignant A/B = 70/30 V/V en 20 minutes et ensuite conservé constant durant 20 min.

Méthode 2 (pour la CLPH préparative) : gradient linéaire commençant avec A/B = 80/20,V/V atteignant A/B = 70/30, V/V en 20 min.

### 2- Synthèse du composé (7) DOX-GAL-AF

### 2a- Préparation du composé (1)

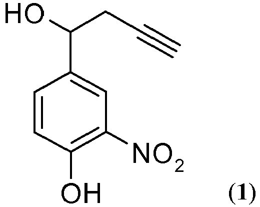

Dans un tricol de 250 mL, 648 mg (24 mmol, 1 éq.) d'aluminium et une quantité catalytique de HgCl₂ sont recouvert de THF préalablement distillé (10 mL). On ajoute 2,6 mL (24 mmol, 1 éq.) de bromure de propargyle pur jusqu'à ce que la réaction démarre (apparition d'une ébullition dans le ballon et noircissement de la solution). Lorsque l'addition est finie, on laissse à reflux pendant 6h. Par la suite, on refroidit la solution à 0°C et on ajoute goutte à goutte une solution composée de 650 mg (3,84 mmol) de 4-hydroxy-3-nitro-benzaldéhyde dans 5mL de THF distillé. Après 30 minutes d'agitation, le produit de départ a totalement disparu, la réaction est alors hydrolysée avec 10 mL de HCl (1N) puis extraite en trois fois avec de l'AcOEt. La phase organique est séchée sur MgSO₄ puis évaporée à sec pour conduire à une huile marron qui est purifié par flash chromatographie (70/30, EP/AcOEt), on obtient une huile jaune qui est diluée dans 30 mL de dichlorométhane. Cette phase organique est lavée trois fois avec NaOH (1N). La phase aqueuse est acidifiée avec HCl concentré et extraite trois fois au chloroforme pour conduire après évaporation au composé **1** sous forme d'une huile marron avec un rendement de 94% (754 mg, 3,6 mmol).
**Formule brute** : C₁₀H₉O₄N et M = 207,19 g/mol
**RMN ¹H (CDCl₃)** δ **(ppm) :** 2,05 (d, 1H, *J* = 2,6 Hz H_{f}) ; 2,58 (dd, 2H, *J* = 6,3 et 2,1 Hz, Hₑ) ; 3,25 (sl, 1H, OH_{benzylique}) ; 4,81 (t, 1H, *J =* 6,2 Hz, H_{d}) ; 7,09 (d, 1H, *J =* 8,7 Hz, Hₐ) ; 7,59 (dd, 1H, *J* = 8,7 et 2,2 Hz, H_{b}) ; 8,07 (d, 1H, *J* = 2,2 Hz, H_{c}) ; 10,48 (sl, 1H, OH_{phénol}).
**RMN ¹³C (CDCl₃) δ (ppm) :** 30,0 (Cₑ) ; 70,9 (C_{d}) ; 72,1 (C_{f}) ; 79,7 (C_{alcyne}) ; 120,5 (C_{a ou b}) ; 122,3 (C_{a ou b}) ; 132,9 (Cₐᵣₒ) ; 133,2 (C_{c}) ; 135,1 (Cₐᵣₒ) ; 154,6 (C_{NO2}).

### 2b- Préparation du composé (2)

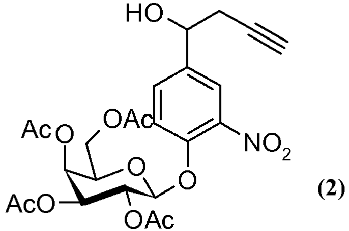

200 µL (0,7 mmol) de HMTTA et 1,0 g d'Ag₂CO₃ (3,7 mmol) sont mis en solution dans 1,5 mL d'acétonitrile. Cette solution est agitée pendant 2h, puis on ajoute successivement 209 mg (1 mmol) de **1** et 830 mg de galactose bromé (2 mmol). Après 4h d'agitation, On additionne de l'eau distillée et on extrait en trois fois avec de l'AcOEt. Les phases organiques sont réunies et lavées avec une solution d'HCl (1N). Après séchage sur MgSO₄ et évaporation à sec, le produit est purifié par flash chromatographie (60/40%, 50/50 et 40/60 EP/AcOEt) pour conduire à 453 mg du produit 2 sous forme d'une huile jaune (0,84 mmol) avec un rendement de 84%.
**Formule brute :** C₂₄H₂₇O₁₃N et M = 537,48 g/mol
**F** : 71°C
**[α]_{D}²⁰:** +42(c 0,1, CHCl₃)
**RMN ¹H (CDCl₃)** δ **(ppm) :** 2,01 (s, 3H, H_{acétate}) ; 2,03 (s, 3H, H_{acétate}) ; 2,05 (s, 4H, H_{acétate} et H_{f}) ; 2,65 (d, 2H, *J* = 4,2 Hz, Hₑ) ; 4,08-4,27 (m, 3H, H_{5,6}) ; 4,92 (t, 1H, *J=* 5,7 Hz, H_{d}) ; 5,07-5,15 (m, 2H, H_{sucre}) ; 5,39-5,56 (m, 2H, H_{sucre}) ; 7,35 (d, 1H, *J* = 8,6 Hz, Hₐ) ; 7,58 (t, 1H, *J* = 8,7 Hz, H_{b}) ; 7,86 (d, 1H, *J* = 8,6 Hz, H_{c}).
**RMN ¹³C (CDCl₃)** δ **(ppm) :** 20,6 (C_{acétate}) ; 20,7 (C_{acétate}) ; 20,8 (C_{acétate}) ; 21,0 (C_{acétate}) ; 29,4 (Ce) ; 61,4 (C₆) ; 66,7 (C_{sucre}) ; 68,9 (C_{sucre}) ; 70,4 (C_{sucre}) ; 71,4 (C_{sucre}) ; 71,9 (C_{f}) ; 79,6 (C_{d}) ; 100,7 (C₁); 119,6 (C_{CHaro}) ; 122,7 (C_{CHaro}) ; 131,1 (C_{CHaro}) ; 138,8 (C_{aro quaternaire}) ; 141,1 (C_{aro quaternaire}) ; 148,7 (C_{aro quaternaire}) ; 169,5 (C_{carbonyle}) ; 170,2 (C_{carbonyle}) ; 170,4 (C_{carbonyle}) ; 171,1 (C_{carbonyle}).

### 2c- Préparation du composé (3)

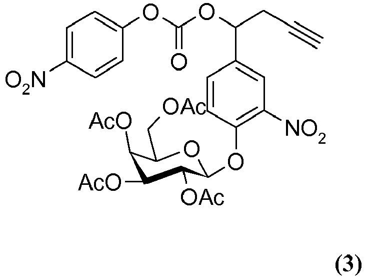

453 mg (0,84 mmol, 1 éq.) d'alcool benzylique **2** et 339 mg (1,68 mmol, 2 éq.) de chloroformate de paranitrophénol sont mis en solution dans 10 mL de DCM. La solution est refroidie à 0°C puis, on additionne 230 µL de pyridine (2,1 mmol, 2,5éq.). Après disparition totale du produit de départ, on additionne de l'eau distillée dans le milieu et on extrait en trois fois au dichlorométhane. La phase organique est séchée sur MgSO₄ puis évaporée à sec. Une flash chromatographie (60/40 et 50/50 EP/AcOEt) permet d'isoler 384 mg du composé **3** (0,55 mmol) sous forme d'un solide jaune avec un rendement de 65 %.
**Formule brute :** C₃₁H₃₀O₁₇N₂ et M = 702,59 g/mol
**F :** 94°C
**[α]_{D}²⁰:** + 32 (c 0,1, CHCl₃)
**RMN ¹H (CDCl₃)** δ **(ppm) :** 2,02 (s, 3H, H_{acétate}) ; 2,05-2,07 (s, 3H, H_{acétate}) ; 2,10 (s, 3H, H_{acétate}) ; 2,15 (s, 3H, H_{acétate}) ; 2,89 (m, 2H, Hₑ) ; 4,11-4,24 (m, 3H, H₅,₆) ; 5,10 (m, 2H, H_{sucre}) ; 5,50 (m, 2H, H_{sucre}) ; 5,81 (t, 1H, *J* = 5,7 Hz, H_{d}) ; 7,34 (d, 2H, *J* = 7,0 Hz, Hg) ; 7,41 (d, 1H, *J =* 8,7 Hz, Hₐ) ; 7,61 (dd, 1H, *J =* 8,7 et 2,1 Hz, H_{b}) ; 7,92 (d, 1H, *J =* 2,1 Hz, H_{c}) ; 8,23 (d, 2H, *J* = 7,1 Hz, Hₕ).
**RMN ¹³C (CDCl₃) δ (ppm) :** 25,2 (C_{acétate}) ; 25,3 (3*C_{acétate}) ; 31,0 (Ce) ; 6,9 (C₆) ; 72,8 (C_{sucre}) ; 73,5 (C_{sucre}) ; 76,0 (C_{sucre}) ; 77,1 (C_{sucre}) ; 78,1 (C_{d}) ; 82,73 (C_{f}) ; 83,9 (C_{alcyne}) ; 105,2 (C₁) ; 123,8 (C_{CH aro}) ; 127,8 (C_{CH aro}) ; 128,8 (C_{CH aro}) ; 130,8 (C_{CH aro}) ; 137,8 (C_{CH aro}) ; 139,0 (Cₐᵣₒ) ; 146,5 (Cₐᵣₒ) ; 151,5 (Cₐᵣₒ) ; 154,8 (Cₐᵣₒ) ; 157,1 (Cₐᵣₒ) ; 161,1 (C_{carbonate}) ; 174,2 (C_{carbonyle}) ; 174,9 (C_{carbonyle}) ; 175,2 (C_{carbonyle}) ; 175,5 (C_{carbonyle}).
**SMHR** : C₃₁H₃₀N₂O₁₇Na(M+Na)⁺ théorique: 725,1442, trouvé : 725,1449
C₃₁H₃₀N₂O₁₇K(M+K)⁺ théorique : 741,1182, trouvé : 741.1186

### 2d- Préparation du composé (4)

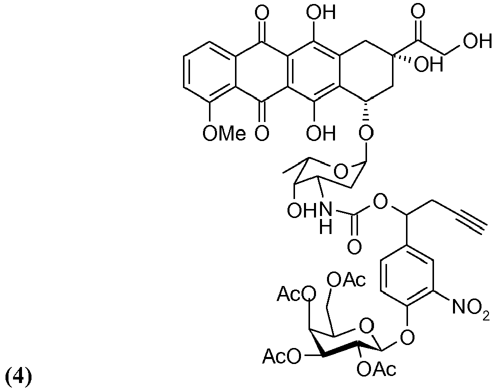

702 mg (1 mmol) de nitrophénol **3** et 580 mg (1 mmol) de doxorubicine hydrochlorée sont mis en solution dans 15 mL de DMF. 350 µL de triéthylamine (2,5 mmol) sont ajoutées dans le milieu. La solution est agitée toute la nuit, puis 25 mL d'une solution saturée de NaCl sont additionnés et le mélange est extrait par trois fois avec du dichlorométhane. La phase organique est séchée sur MgSO₄ puis évaporée. Le produit est purifié par flash chromatographie (1,5/98,5, 3/97 et 4/96 MeOH/DCM) sur gel de silice. On isole le composé **4** sous forme d'un solide rouge avec un rendement de 50% (554 mg, 0,50 mmol).
**Formule brute :** C₅₂H₅₄O₂₅N₂ et M = 1107,02 g/mol
**F:** 172°C
**[α]_{D}²⁰:** +180 (c 0,1, CHCl₃)
**RMN ¹H (CDCl₃)** δ **(ppm) :** 1,25 (m, 3H, H_{Dox 6'}) ; 1,81-2,38 et 2,68-3,23 (m, 23H) ; 3,47 (s, 1H) ; 3,58-3,83 (m, 2H) ; 4,07-4,21 (m, 7H) ; 4,51 (s, 1H) ; 4,74 (m, 2H) ; 5,25 (sl, 1H) ; 5,47 (m, 4H) ; 5,68 (m, 1H) ; 7,38 (d, 1H, *J* = 8,4 Hz, Hₐᵣₒ) ; 7,48 (dd, 1H, *J=* 8,7 et 2,1 Hz, Hₐᵣₒ) ; 7,77 (m, 2H, Hₐᵣₒ) ; 7,99 (dd, *J* = 7,3 et 2,3 Hz, Hₐᵣₒ) ; 13,15 (d, 1H, OH_{phénol}) ; 13,92 (d, 1H, OH_{phénol}).
**RMN ¹³C (CDCl₃) δ (ppm) :** 16,8 ; 20,6 (2*) ; 26,4 (2*) ; 31,5 ; 33,8 ; 35,6 ; 36, 5 ; 47,2 ; 56,6 ; 61,3 ; 65,5 ; 66,7 ; 67,4 ; 67,8 ; 69,4 ; 69,5 ; 69,6 ; 70,5 ; 71,4 ; 71,8 ; 72,4 ; 72,5 ; 78,5 (2*) ; 100,5 ; 111,2 ; 111,4 ; 118,6 ; 119,3 ; 119,7 ; 120,6 ; 123,3 ; 131,8 ; 133,6 ; 135,3 ; 135,4 ; 135,8 ; 140,9 ; 149,1 ; 155,5 ; 156,1 ; 161,0 ; 162,7 ; 169,4 ; 170,1 ; 170,2 ; 170,3 (2*) ; 186,5 ; 186,9 ; 213,8.
**SMHR :** C₅₂H₅₄N₂O₂₅Na(M+Na)⁺ théorique : 1129,2913, trouvé : 1129,2897
C₅₂H₅₃N₂O₂₅Na₂ (M-2H+2Na)⁺ théorique : 1151,2733, trouvé : 1151,2726

### 2e- Préparation du composé (5) DOX-GAL

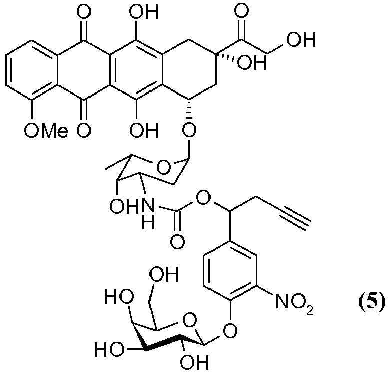

104 mg (94µmol, 1éq.) de prodrogue **4** est dilués dans 34 mL de MeOH et 6 mL de DCM. La solution est refroidie à -15°C à l'aide d'un cryostat. On additionne dans le milieu 79 mg de MeONa (1,5 mmol, 16 éq.). La solution est agitée entre 2h15 et 2h30 à -15°C, puis après disparition totale du produit de départ, le milieu est neutralisé avec une résine acide (IRC 50) pendant 20 minutes. Le mélange est filtré sur coton, rincé au MeOH, puis évaporé à sec. Une purification par flash chromatographie (6/94, 10/90 et 15/85 MeOH/DCM) permet d'isoler 71 mg du produit **5** (75 µmol) sous forme d'un solide rouge avec un rendement de 80%.
**Formule brute :** C₄₄H₄₈O₂₁N₂ et M = 940,87 g/mol
**F:** 131°C
**[α]_{D}²⁰ :** +128 (c 0,05, MeOH)
**RMN ¹H (MeOD) δ (ppm)** : 1,26 (m, 3H, H_{6'}) ; 1,82 (m, 1H, H_{Dox 2'}) ; 1,87-2,15 (m, 2H, H_{Dox 8a/b} et H_{Dox 2'}) ; 2,25 (m, 2H, H_{f} et H_{8a/b}) ; 2,68 (m, 3H, Hₑ et H_{Dox 10a/b}) ; 2,91 (m, 1H, H_{Dox 10a/b}) ; 3,53-3,89 (m, 11H, H₂,₃,₄,₅,₆,_{3'},_{4'},_{OMe}) ; 4,20 (m, 1H, H_{5'}) ; 4,70 (m, 2H, H₁₄) ; 4,87 (m, 1H, H_{Dox 7}) ; 5,0 (d, 1H, *J* = 7,7 Hz, H₁) ; 5,33 (m, 1H, H_{1'}) ; 5,65 (dt, 1H, H_{d}) ; 7,22 (d, 0,5 H, *J* = 8,8Hz, Hₐ) ; 7,31 (m, 1H, H_{Dox 3}) ; 7,41 (d, 0,5H, *J=* 8,7 Hz, Hₐ) ; 7,50-7,63 (m, 3H, H_{Dox 1,2} et H_{b}) ; 7,75 (d, 0,5H, *J* = 1,9 Hz, H_{c}) ; 7,82 (d, 0,5H, *J* = 2,0 Hz, H_{c}).
**RMN ¹³C (MeOD) δ (ppm) :** 16,1 ; 17,2 ; 26,0 ; 29,6 ; 32,9 ; 36,1 ; 55,9 ; 61,2 ; 64,6 ; 66,4 ; 69,0 ; 70,8 ; 71,5 ; 71,8 ; 72,9 ; 73,7 ; 76,2 ; 76,8 ; 78,9 ; 98,2 ; 101,9 ; 117,6 ; 119,0 ; 119,2 ; 131,9 ; 134, 4 ; 135,0 ; 136,1 ; 140,6 ; 150,0 ; 155,1 ; 155,7 ; 161,1 ; 186,3 ; 214,0.
**SMHR** : C₄₄H₄₆N₂O₂₁Na(M+Na)⁺ théorique : 961,2491, trouvé : 961,2497
C₄₄H₄₅N₂O₂₁Na₂(M-H+2Na)⁺ théorique : 983,2310, trouvé : 983,2276

### 2f- Préparation du composé (6)

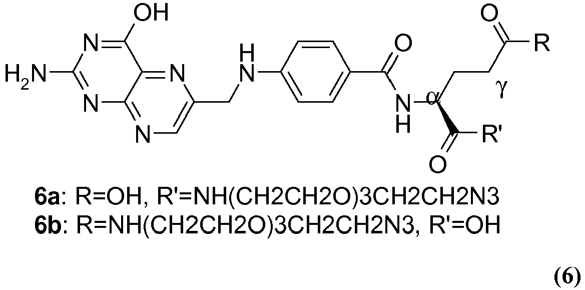

A une solution d'acide folique dihydraté (600 mg, 1,26 mmol) dans du DMSO (20 ml) et de la pyridine (10 ml) ont été ajoutés le 2-(2-(2-(2-azidoethoxy)ethoxy)ethoxy)ethanamine (0,3 ml, 1,2, equiv.) (commercialement disponible ou préparé comme décrit par Schwabacher et al., J. Org. Chem. 1998, 63 : 1727) et du dicyclohexylcarbodiimide (DCC) (650 mg, 2,5 equiv.). Le mélange réactionnel a été agité pendant la nuit, dans le noir, à température ambiante, pour conduire à la formation d'un précipité de dicyclohexylurée (DCU). Après élimination du précipité, le filtrat a été versé dans une solution d'éther anhydre (400 ml) refroidit à 0°C. Le précipité jaune ainsi obtenu a été récupéré par filtration, puis lavé avec Et₂O pour éliminer les traces de DMSO. Une analyse CLHP du précipité (Méthode 1) a montré que les deux isomères **6a** et **6b** ont été obtenus dans un rapport de 7 / 3. Le solide (812 mg) a été dissout dans une solution (8 ml) 8:2:0,3:0,3 H₂O/ACN/DMSO/Et₃N, et purifié par une CLHP préparative en phase inverse (Méthode 2). La fraction contenant les isomères **6a** et **6b** inséparables a été concentrée sous pression réduite. Le résidu a été ensuite dissout dans du méthanol à reflux et versé dans une solution de Et₂O refroidie à 0°C. Le précipité a été récupéré par filtration et lavé avec Et₂O pour donner une poudre jaune (240 mg, 30 %).
**RMN ¹H (DMSO-*d₆*) δ (ppm)** : 8.69 (s, 1H), 7.99 (d, 1H, *J* = 8.3Hz), 7.89 (t, 1H, *J* = 5.6 Hz), 7.68 (d, 2H, *J=* 9.7 Hz), 7.19 (bs, 1H), 6.66 (d, 2H, *J=* 9.7 Hz), 4.53 (s, 2H), 4.4 (m, 1H), 3.72-3.19 (m, 20H), 2.30-2.20 (m, 2H), 2.11-1.82 (m, 2H)
**RMN ¹³C (DMSO-*d₆*) δ (ppm) :** 174.1, 171.8, 166.7, 154.9, 153.5, 150.7, 149.2, 148.4, 129.1, 127.9, 121.2, 69.8, 69.7, 69.6, 69.5, 69.3, 68.4, 52.6, 50.0, 48.6, 45.9, 43.6, 38.5, 30.5, 27.1
**SMHR:** C₂₇H₃₄N₁₁O₈ (M-H)⁻ théorique : 640.2593, trouvé : 640.2586

### 2g- Préparation du composé (7) (DOX-GAL-AF)

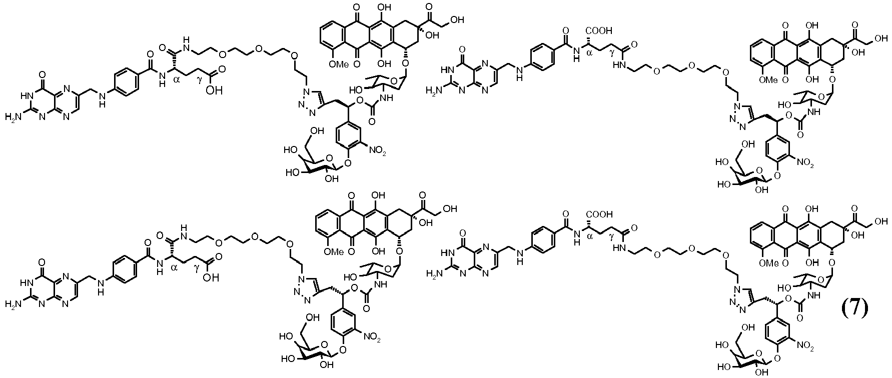

A une solution du composé **(5)** (25 mg, 26 µmol) et du composé **(6)** (16 mg, 0,95 equiv.) dans du DMSO (1,5 ml) contenant 10 % d'eau ont été ajoutés de l'ascorbate de sodium (10,5 mg, 2 equiv.) et du CuSO₄ (4 mg, 0,6 equiv.). La solution a été agitée à température ambiante et la réaction a été suivie par CLHP (Méthode 1). Trois fractions de CuSO₄ (2 mg, 0,3 equiv.) ont été ensuite ajoutées régulièrement jusqu'à disparition du composé (6). Après 6 heures, le mélange réactionnel a été dilué avec du MeOH (2 ml) et versé dans une solution d'éther refroidie à 0°C. Le précipité ainsi obtenu a été filtré et lavé avec MeOH et Et₂O pour donner une poudre de couleur pourpre (40 mg). Ce solide a été dissout à 0 °C dans du DMSO (1 ml) et une solution d'EDTA. 2Na.2H₂O (56 mg, 6 equiv.) dans du tampon phosphate (1 ml, 0,2 M, pH 7). La solution a été agitée à température ambiante pendant 5 heures. La couleur vire alors au rouge. La solution a été filtrée, et le filtrat a été dilué avec du MeOH et versée dans l'éther à 0°C. Le précipité obtenu a alors été filtré et lavé avec MeOH et Et₂O pour donner le composé (7) (30 mg, 75 %) sous forme d'une poudre rouge. L'analyse CLHP (Méthode 1) a montré quatre pics (temps de rétention : 23,7 min, 24,4 min, 25,3 min et 26,1 min) correspondant aux quatre isomères du composé (7) (DOX-GAL-AF).
**RMN ¹H (DMSO-*d₆*) δ (ppm) :** 8.63 (s, 1H), 7.90 (m, 5H), 7.65 (m, 4H), 7.6 (m, 2H), 7.35 (m, 2H), 6.91 (m, 2H), 6.64 (m, 2H), 5.76 (m, 1H), 5.21 (m, 1H), 4.94 (m, 3H), 4.56 (s, 3H), 4.56-4.33 (m, 7H), 4.14-3.99 (m, 7H), 2.97(m, 3H), 2.70 (m, 1H), 2.35 (m, 1H), 2.23-2.13 (m, 4H), 2.10-1.85 (m, 4H), 1.45 (m, 1H), 1.12 (m, 3H)
**SMHR :** C₇₁H₈₀N₁₃O₂₉ (M-H)⁻ théorique : 1578.51904, trouvé : 15785115

### Exemple 2

### Sélectivité du composé (7) DOX-GAL-AF

Le composé **(7)** a été testé sur deux lignées cellulaires, les cellules Hela, exprimant le récepteur de l'acide folique (FR), et les cellules A549 exprimant peu ou pas ces récepteurs.

Les cellules Hela et A549 ont été cultivées dans un milieu RPMI 1640 supplémenté avec du sérum de veau foetal à 10 % (InVitrogen), à 37 °C dans une atmosphère à 5 % de CO₂.

Pour les expériences en microscopie confocale, les cellules ont été cultivées en cuvette à fond de verre.

Les cellules ont été incubées pendant 1 heure en présence ou en absence de 1 µm d'acide folique avant d'être traitée, avec soit une solution tampon (témoin), soit de la doxorubicine à 10 µM, soit avec un conjugué doxorubicine-galactose (DOX-GAL), composé **(5),** à la concentration de 10 µM, soit le composé (7) (DOX-GAL-AF) à la concentration de 10 µM, soit le composé **(7)** en présence d'acide folique aux concentrations respectives de 10 µM.

Après traitement, les cellules ont été rincées avec du PBS et fixées avec une solution PBS-formaldéhyde 3,7% pendant 20 minutes, à température de la pièce.

Les cellules fixées ont été rincées avec du PBS, puis montées avec un milieu Vectashield Médium en présence de DAPI (Victor Laboratories Inc., Abcys, Paris France) avant observation au microscope confocale (FV 1000, Olympus IX-81, Tokyo, Japan). Les longueurs d'onde d'excitation de 405 nm et 488 nm ont été respectivement utilisée pour l'acquisition des images en bleu (450 nm) pour le DAPI, et en rouge (590 nm) pour la doxorubicine.

L'internalisation par endocytose de la doxorobucine des composés **(5)** et **(7)** et du composé (7) en présence d'acide folique (AF) a été suivie par microscopie confocal grâce à la fluorescence de la doxorubicine dans les cellules Hela et dans les cellules A549.

Les résultats obtenus au cours de cette étude sont regroupés sur la Figure 9.

Les résultats montrent que le composé (7) DOX-GAL-AF n'est internalisé que dans les cellules Hela, alors que la doxorubicine (DOX) pénètre de manière non spécifique, à travers les membranes des deux lignées cellulaires et que le composé **(5)** DOX-GAL n'est internalisé dans aucune des lignées cellulaires.

De plus, lorsque le milieu de culture a été préalablement saturé avec de l'acide folique, la fluorescence intracellulaire de la doxorubicine dans les cellules Hela incubées en présence du composé **(7)** DOX-GAL-AF est nettement atténuée.

L'ensemble de ces résultats démontre que le composé **(7)** DOX-GAL-AF permet l'internalisation sélective et spécifique de la doxorubicine dans les cellules cibles exprimant le récepteur de l'acide folique.

### Exemple 3

### Toxicité du composé (7) DOX-GAL-AF

Afin de vérifier que l'endocytose du composé **(7)** DOX-GAL-AF est suivie par la libération de la drogue dans le milieu intracellulaire, des tests de prolifération ont été réalisés sur des cellules Hela et A549 incubées en présence de concentrations croissantes (de 0 à 1000 nM) de doxorubicine (DOX), de composé **(5)** (DOX-GAL) et de composé **(7)** (DOX-GAL-AF) pendant 4 jours.

Le kit « cell proliferation kit II » (XTT ; Roche) a été utilisé pour évaluer la prolifération cellulaire. Le kit a été utilisé selon les recommandations du fabricant.

En résumé, 3x10³ cellules/puits ont été mis en plaques de culture 96 puis dans 100 µl de milieu.

Les cellules sont cultivées comme indiqué précédemment.

Les cellules sont cultivées pendant 24 heures avant addition des composés testés à la concentration indiquée.

Après 4 jours de traitement, 50 µl d'un mélange de marquage XTT ont été ajoutés au puits. Les cellules ont été ensuite incubées pendant 4 heures à 37 °C avant que l'absorbance ne soit déterminée à 480 nm.

Les résultats, illustrés par la Figure 3 (A et B) montrent que le composé (7) DOX-GAL-AF, triangle noir, trait continu, inhibe la prolifération des cellules Hela (0,1 µM < IC₅₀ < 0,2 µM) (Figure 3A), alors qu'il ne présente pas de toxicité vis-à-vis des cellules A549 (Figure 3B).

En revanche, le composé **(5)** (DOX-GAL), carré noir, trait continu, est inefficace sur les deux lignées cellulaires, et la doxorubicine, rond noir, inhibe la prolifération de ces deux lignées.

Afin de vérifier que l'activation enzymatique du composé **(7)** DOX-GAF-AF peut effectivement se produire dans l'environnement cellulaire, les tests de cytotoxicité ont été répétés dans un milieu de culture préalablement supplémenté en β-galactosidase (E. coli) (40 U.mL⁻¹) (courbes en pointillées).

Dans ce cas, le composé **(7)** exerce une activité antiproliférative similaire sur les deux lignées cellulaires (HeLa exprimant le récepteur de l'acide folique et A589 n'exprimant pas ce récepteur) la même activité antiproliférative.

La présente invention concerne donc notamment, un nouveau bras auto-réactif, de nouvelles prodrogues comprenant un tel bras conçues pour traiter sélectivement les tissus cibles. En particulier, l'invention concerne de nouveaux agents anticancéreux conçus pour détruire sélectivement les tumeurs sans affecter les organes sains.

La présente invention permet, avantageusement, un ciblage thérapeutique ou diagnostique amélioré, et plus efficace, au travers de l'utilisation de prodrogues capables d'être adressées spécifiquement aux cellules cibles et au microenvironnement des cellules cibles.

Plus particulièrement, l'invention concerne de nouvelles prodrogues anticancéreuses capables de cibler d'une part les cellules tumorales et d'autre part le microenvironnement des cellules tumorales.

La présente invention concerne également un procédé de synthèse de nouvelles prodrogues s'appuyant sur une réactivité spécifique du bras auto-réactif de l'invention. Le procédé de synthèse de l'invention permet d'accéder de manière simple et efficace à un large éventail de prodrogues conforme à l'invention.

Les prodrogues de l'invention ne présentent avantageusement pas d'effet à l'égard des cellules ou des tissus non ciblés.

### Exemple 4

### Synthèse du vecteur dendritique (221)

### Préparation du composé 227 :

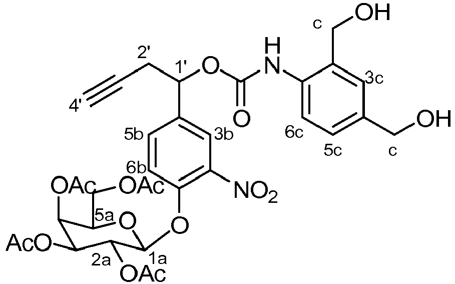

Le carbonate mixte **222** (100 mg, 0,142 mmol) et l'aniline **117** (43,6 mg, 0,285 mmol, 2 éq.) sont dilués dans 2 mL de DMF. On additionne dans le milieu HOBt (19,2 mg, 0,142 mmol, 1éq.). La solution est agitée 3h à 50°C. Le DMF est alors évaporé sous vide. Une purification par flash chromatographie (50/50, 75/25 AcOEt/EP) permet d'isoler le produit **227** sous forme d'une mousse blanche avec un rendement de 98% (100 mg, 0,139 mmol).
**Formule brute :** C₃₃H₃₆N₂O₁₆
**M** = 716,62 g/mol
**Rf** = 0,5 (AcOEt)
**RMN ¹H (400 MHz, CDCl₃) δ (ppm) :** 2,00 (s, 3H, CH₃COO) ; 2,04-2,1 (m, 7H, 2* CH₃COO, H_{4'}) ; 2,17 (s, 3H, CH₃COO) ; 2,65 (sl, 1H, CH₂OH) ; 2,78 (m, 2H, H_{2'}) ; 3,15 (sl, 1H, CH₂OH) ; 4,15 (m, 3H, H₅ₐ, H₆ₐ) ; 4,53 (s, 2H, H_{c}) ; 4,61 (s, 2H, H_{c}) ; 5,09 (m, 2H, H₃ₐ, H₁ₐ) ; 5,44 (d, 1H, *J_{H4a-H3a}* = 3,0 Hz, H₄ₐ) ; 5,51 (dd, 1H, *J_{H2a-H3a}* = 11,0 Hz, *J_{H2a-H1a}* = 8,0 Hz, H₂ₐ) ; 5,82 (t, 1H, *J_{H1'-H2'}* = 6,5 Hz, H_{1'}) ; 7,09 (d, 1H, *J_{H3c-H5c}* = 1 Hz, H_{3c}) ; 7,18 (d, 1H, *J_{H5c-H6c}* = 8,3 Hz, H_{5c}) ; 7,34 (d, 1H, *J_{H6b-H5b}* = 8,6 Hz, H_{6b}) ; 7,58 (d, 1H, *J_{H5b-H6b}* = 8,6 Hz, H_{5b}) ; 7,75 (d, 1H, *J_{H6c-H5c}* = 8,3 Hz, H_{6c}). 7,88 (s, 1H, H_{3b}) ; 8,19 (sl, 1H, NH)
**RMN ¹³C (100 MHz, (CD₃)OD) δ (ppm) :** 20,5-20,6 (4*OCOCH₃) ; 26,3-26,39 (C₂, dia) ; 61,3 (C₆ₐ) ; 63,9 (C_{c}) ; 64,4 (C_{c}) ; 66,7 (C₄ₐ) ; 67,9 (C₂ₐ) ; 70,5 (C₃ₐ) ; 71,4 (C₅ₐ) ; 71,9 (C_{4'}) ; 72,9 (H_{1'}) ; 78,51 (C_{3'}) ; 100,5 (C₁ₐ) ; 119,4 (C_{6b}) ; 121,0 (C_{6b}) ; 123,2-123,4 (C_{3b} dia) ; 127,5 (C_{5c}) ; 127,6 (C_{3c}) ; 129,6 (C_{4b}) ; 132,0-132,3 (C_{5b} dia) ; 135,1 (C_{2b}) ; 136,3-136,4 (C_{5b} dia) ; 140,9 (C_{2c}) ; 141,0 (C_{4c}) ; 149,2-149,0 (C_{1c} dia) ; 152,7 (C_{1b}) ; 169,5-170,5 (4*CO_{acétate})
**SMHR (ESI)** : C₃₃H₃₆N₂O₁₆Na [M+Na]⁺ m/z théorique : 739,19625 m/z trouvé : 739,1961
C₃₃H₃₆N₂O₁₆K [M+K]⁺ m/z théorique : 755,17019 m/z trouvé : 755,1684

### Préparation du composé 228 :

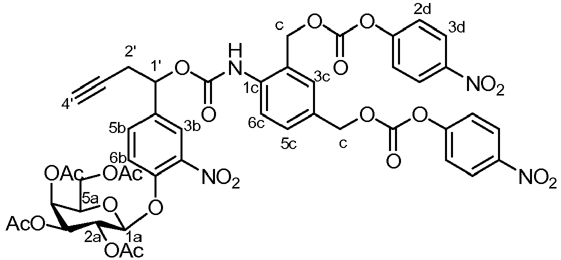

Le dialcool **227** (335 mg 0,47 mmol), en solution dans 3 mL de DCM est additionné sur une solution de chloroformiate de para-nitrophényle (376 mg, 1,87 mmol, 4éq.) et de pyridine (151,3 µL, 1,87 mmol, 4 éq.) dans 6 mL de DCM à 0°C. Après 1 heure d'agitation, le brut réactionnel est hydrolysé par une solution saturée de NaCl puis extrait 3 fois au DCM. Le brut réactionnel est alors purifié par flash chromatographie (éluant : MeOH/DCM : 0/1 et 1/99) pour conduire au composé **228** sous forme d'une mousse blanche avec un rendement de 89% (441 mg, 0,42 mmol).
**Formule brute :** C₄₇H₄₂N₄O₂₄
**M** = 1046,84 g/mol
**Rf** = 0,5 (60/40 : AcOEt/EP)
**RMN ¹H (400 MHz, CDCl₃) δ (ppm) :** 2,02 (s, 3H, CH₃COO) ; 2,06-2,12 (m, 7H, 2* CH₃COO, N_{4'}) ; 2,19 (s, 3H, CH₃COO) ; 2,83 (m, 2H, H_{2'}) ; 4,20 (m, 3H, H₅ₐ, H₆ₐ) ; 5,12 (m, 2H, H₃ₐ, H₁ₐ) ; 5,28 (s, 2H, H_{c}) ; 5,34 (s, 2H, H_{c}) ; 5,48 (d, 1H, *J_{H4a-H3a}* = 3,4 Hz, H₄ₐ) ; 5,55 (dd, 1H, *J_{H2a-H3a}* = 10,0 Hz, *J_{H2a-H1a}* = 8,0 Hz, H₂ₐ) ; 5,88 (t, 1H, *J_{H1'-H2'}* = 6,5 Hz, H_{1'}) ; 7,37 (m, 5H, 5H, 4*H_{2d}, H_{6b}) ; 7,49 (dd, 1H, *J_{H5c-H6c}* = 8,3 Hz, *J_{H5c-H3c}* = 1,8 Hz, H_{5c}) ; 7,51 (d, 1H, *J_{H3c-H5c}* = 1,8 Hz, H_{3c}) ; 7,59 (dd, 1H, *J_{H5b-H6b}* = 8,7 Hz, *J_{H5b-H3b}* = 1,6 Hz, H_{5b}) ; 7,68 (sl, 2H, NH_{carbamate}) ; 7,84 (m, 1H, H_{6c}) ; 7,91 (s, 1H, H_{3b}) ; 7,26 (m, 4H, 4*H_{3d})
**RMN ¹³C (100 MHz, CDCl₃) δ (ppm) :** 20,5-20,6-20,7 (4* C_{acétate}) ; 26,3 (C_{2'}) ; 61,3 (C₆ₐ) ; 66,7 (C₄ₐ) ; 67,5 (C_{c}) ; 67,8-67,8 (C₂ₐ dia) ; 70,0 (C_{c}) ; 70,5 (C₃ₐ) ; 71,4 (C₅ₐ), 72,0 (C_{4'}) ; 73,3 (C₂ₐ) ; 78,3-78,4 (C_{3'} dia) ; 100,5-100,6 (C₁ₐ dia) ; 119,3-119,5 (C_{6b} dia) ; 121,8 (4*C_{2d}) , 123,2 (C_{3b}) ; 123,3 (C_{6c}) ; 125,3-125,4 (4*C_{3d}) ; 130,9 (C_{5c}) ; 131,7 (C_{3c}) ; 132,1-132,2 (C_{5b}) ; 134,8-134,8 (2*C_{4d}) ; 137,0 (C_{1c}) ; 141,0-141,1 (2*C_{1d}) ; 145,4 (C_{4b}) ; 145,6 (C_{2b}) ; 149,3 (C_{4c}) ; 152,4 (C_{2c}) ; 152,6 (C_{1b}) ; 152,9 (CO_{carbamate}) ; 155,1-155,4 (2*CO_{carbonate}) ; 169,3-170,1-170,2-170,3 (4*CO_{acétate})
**SMHR (ESI) :** C₄₇H₄₂N₄O₂₄Na [M+Na]⁺ m/z théorique : 1069,20812 m/z trouvé : 1069,2081
C₄₇H₄₂N₄O₂₄K [M+K]⁺ m/z théorique : 1085,18206 m/z trouvé : 1085,1810

### Préparation du composé 229 :

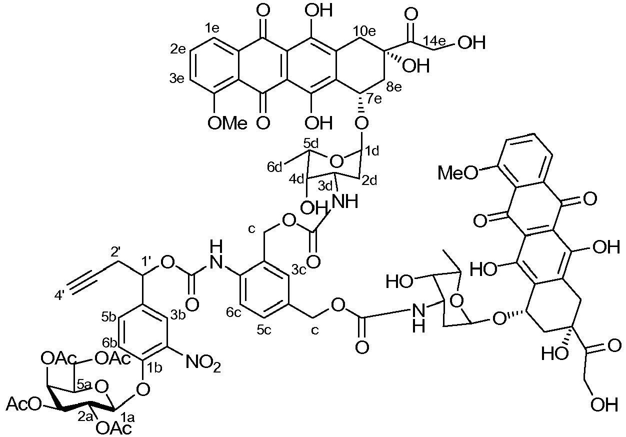

La doxorubicine chlorohydrate (119,7 mg, 0,206 mmol, 2 éq.) est mise en solution dans 1 mL de DMF. On additionne la triéthylamine (28,7 µL, 0,206 mmol, 2 éq.) et on laisse agiter à température ambiante pendant 20 minutes. On ajoute ensuite HOBt (27,8 mg, 0,206 mmol, 2 éq.) puis une solution de carbonate **228** (108 mg, 0,103 mmol) dans 1 mL de DMF. Après 3 heures d'agitation à température ambiante, le brut réactionnel est hydrolysé par une solution saturée de NaCl puis extrait 4 fois au DCM et 1 fois à l'AcOEt. Une purification par flash chromatographie (0/1, 1/24 : MeOH/DCM) permet d'isoler le produit **229** sous forme d'une poudre rouge avec un rendement de 62%. (119 mg, 0,064 mmol)
**Formule brute :** C₈₉H₉₀N₄O₄₀
**M** = 1854,51 g/mol
**Rf =** 0,2 (7/93 : MeOH/DCM)
**Point de fusion :** décomposition 215 °C
**RMN ¹H (400 MHz, DMSO-*d6*)** δ **(ppm) :** 1,19 (d, 6H, *J_{H6d-H5d}* =6,1 Hz, 2*H_{6d}) ; 1,57-1,61 (m, 2H, H_{2d}) ; 1,85-1,91 (m, 2H, H_{2d}) ; 2,01-2,27 (m, 17H, 4* CH₃COO, H_{2'}, H_{4'}, 2*H₈ₑ) ; 2,88-2,98 (m, 6H, 2*H₁₀ₑ, H_{2'}) ; 3,51 (m, 1H, 2*H_{4d}) ; 3,75 (m, 2H, 2*H_{3d}) ; 3,97 (2*s, 6H, 2*OMe) ; 4,19 (m, 4H, H₆ₐ, 2*H_{5d}) ; 4,53 (t, 1H, *J_{H5a-H6a}* = 6,1 Hz, H₅ₐ) ; 4,65 (2*s, 4H, 2*H₁₄ₑ) ; 4,74-4,79 (m, 2H, 2*H₇ₑ, 4,92-5,09 (m, 7H, 2*H_{c}, 3*OH) ; 5,27-5,34 (m, 3H, H₂ₐ, H₃ₐ, OH) ; 5,42 (m, 1H, H₄ₐ) 5,46 (sl, 1H, OH) ; 5,48 (sl, 1H, OH) ; 5,65 (d, 1H, *J_{H1a-H2a}* = 7,1 Hz, H₁ₐ) ; 5,84 (m, 1H, H_{1'}) ; 6,88 (d, 1H, *J_{NH-H3d}* = 7,7 Hz, NH_{carbamate}) ; 6,97 (d, 1H, *J_{NH-H3d}* = 7,7 Hz, NH_{carbamate}) ; 7, 25 (d, 1H, *J_{H5c-H6c}* = 8,4 Hz, H_{5c}) ; 7,33-7,36 (m, 2H, H_{6b}, H_{3c}) ; 7,45 (dd, 1H, *J_{H5b-H6b}* = 8,1 Hz, *J_{H5b-H3b}* = 3,4 Hz, H_{5b}) ; 7,63 (m, 2H, 2*H₁ₑ) ; 7,80 (d, 1H, *J_{H6c-H5c}* = 8,5 Hz, H_{6c}) ; 7,84-7,92 (m, 4H, 2*H₁ₑ, 2*H₂ₑ) ; 7,99 (s, 1H, H_{3b}) ; 9,32 (sl, 1H, NH_{carbamate}) ; 13,24 (2*s, 2H, OH_{phénol}) ; 13,97 (2*s, 2H, OH_{phénol})
**RMN ¹³C (100 MHz, DMSO-*d6*)** δ **(ppm) :** 16,9 (2*C_{6d}) ; 20,2-20,3-20,4-20,5 (C_{acétate}) ; 25,4 (C_{2'}) ; 29,7 (2*C_{2d}) ; 32,1 (2*C₁₀ₑ) ; 36,5 (2*C₈ₑ) ; 47,1 (2*C_{3d}) ; 56,4-56,4 (OMe) ; 61,2 (C₆ₐ) ; 63,6 (2*C₁₄ₑ) ; 64,7 (2*C_{c}) ; 66,6 (2*C_{5d}) ; 67,0-67,6-67,8-69,7-69,8-70,7-73,8-74,9-75,0 (2*C_{4d}, C₂ₐ, C₃ₐ, C₄ₐ, C₅ₐ, 2*C₇ₑ, C_{1'}, C_{4'}, 2*C₉ₑ) ; 79,6 (C_{3'}) ; 98,4 (C₁ₐ) ; 100,3 (2*C_{1d}) ; 110,4-110,6 (C_{quaternaire}) ; 117,5 (C_{6b}) ; 118,8 (C_{6c}) ; 119,5-119,9 (2*C₃ₑ) ; 122,5 (C_{3b}) ; 124,3 (C_{5c}) ; 127,6 (C_{5b}, C_{5c}) ; 130,2 (C_{quaternaire}) ; 132,0 (C_{6c}) ; 133,0-134,4-134,8-135,4-135,5 (C_{quaternaire}) ; 136,0 (2*C₂ₑ, 2*C₁ₑ) ; 139,9-147,9 (C_{quaternaire}) ; 153,2-154,4-155,1-156,0-160,6 (3*CO_{carbamate}, 4*C_{phénol}) ; 168,8-169,5-169,8-169,9 (4*CO_{acétate}) ; 186,1-186,3 (4*CO_{quinone}) ; 213,7-213,8 (CO_{cétone})
SMHR (ESI) : C₈₉H₉ₒN₄O₄₀Na [M+Na]⁺ m/z théorique : 1877,5029 m/z trouvé : 1877,5048

### Préparation du composé 225 :

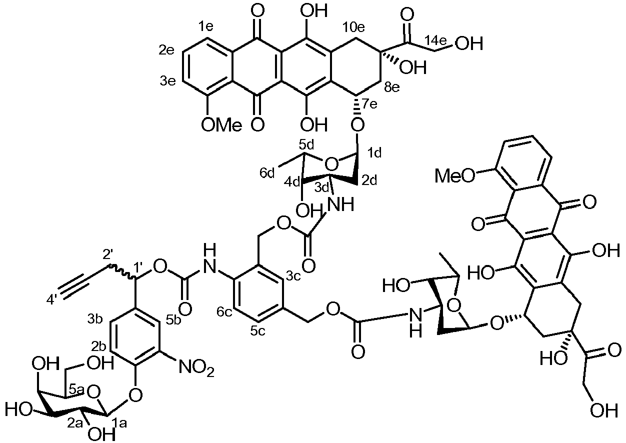

Le composé protégé **229** (156 mg, 84 µmol) est mis en solution dans 6 mL de DCM et 10 mL de MeOH. On additionne du méthanolate de sodium (72,6 mg, 1,34 mmol, 16 éq.) sous agitation à - 15 °C. On laisse agiter à -10 °C pendant 5 heures. Après disparition totale du composé de départ, le milieu est neutralisé avec une résine acide (IRC 50) pendant 15 minutes. Le mélange est filtré sur coton, rincé au MeOH, puis évaporé à sec. Une purification par flash chromatographie (5/95, 10/90 MeOH/DCM) permet d'isoler le produit **225** (115 mg, 68 µmol) sous forme d'un solide rouge avec un rendement de 81%.
**Formule brut :** C₈₁H₈₂N₄O₃₆
**M** = 1686,47 g/mol
**Rf** = 0,2 (15/85 : MeOH/DCM)
**Point de fusion :** décomposition 185 °C
**RMN ¹H (400 MHz, DMSO-*d6*) δ (ppm) :** 1,11 (s, 6H, 2*H_{6d}) ; 1,22-1,87 (m, 7H, H_{2'}, H_{4'}, 2*H_{2d}) ; 1,98-2,21(m, 4H, 2*H₈ₑ) ; 2,81-2,96 (m, 4H, 2*H₁₀ₑ) ; 3,37 (m, 4H, H₂ₐ-H₃ₐ-H₄ₐ-H₅ₐ, masqué par HOD pic résiduel du solvant) ; 3,65 (m, 6H, 2*H_{3d}, 2*H_{5d}, 2*H₇ₑ) ; 3,90 (s, 6H, 2*OMe) ; 4,13 (m, 3H, 2*H_{4d}, OH) ; 4,57 (m, 4H, 2*H₁₄ₑ) ; 4,70 (sl, 2H, 2*OH) ; 4,85-5,06 (m, 9H, 2*CH_{2carbamate}, H_{1'}, H₆ₐ, 2*OH) ; 5,14-5,32 (m, 4H, H₁ₐ, 2*H_{1d}, OH), 5,40-5,54 (m, 4H, 4*OH) ; 6,83 (d, 1H, *J_{NH-H3d}* = 8,0 Hz, NH_{carbamate}) ; 6,93 (d, 1H, J_{NH-H3d} = 8,0 Hz, NH_{carbamate}) ; 7,20 (d, 1H, *J_{H5c-H6c}* = 10,0 Hz, H_{5c}) ; 7,25 (s, 1H, H_{3c}) ; 7,35 (m, 1H, H_{6c}) ; 7,54 (d, 2H, *J_{H1e-H2e}* = 8,0 Hz, 2*H₁ₑ) ; 7,63 (d, 1H, *J_{H6b-H5b}* = 8,0 Hz, H_{6b}) ; 7,75-7,90 (m, 6H, H_{3b}, H_{6b}, 2*H₂ₑ, 2*H₃ₑ) ; 9,08 (sl, 1H, NH_{carbamate}) ; 13,15 (s, 2H, 2*H_{phénol}) ; 13,88 (s, 2H, 2*H_{phénol})
**RMN ¹³C (100 MHz, DMSO-*d6*) δ (ppm) :** 16,9 (2*C_{6d}) ; 29,4-31,5 (C_{2'}-2*C_{2d}) ; 34,3 (2*C₁₀ₑ) ; 36,5 (2*C₈ₑ) ; 47,1 (2*C_{3d}) ; 51,9 (2*C₇ₑ) ; 56,4 (2*COMe) ; 61,5-63,6-64,7-66,5-67,8 (C₆ₐ, C_{1'}, 2*C₁₄ₑ, C_{4'}) ; 69,5-71,1-72,7-74,8-74,9-75,1-75,6 (C₂ₐ, C₃ₐ, C₄ₐ, C₅ₐ, C_{3'}, CH_{benzylique}, 2*C_{4d}, 2*C₉ₑ) ; 99,7-100,2 (C₁ₐ- 2*C_{1d}) ; 110,4-110,5 (C_{quaternaire}) ; 116,8 (C_{6b}) ; 118,7-118,8-119,5-119,7 (2*C₁ₑ-2*C₃ₑ-C_{quaternaire}) ; 124,1-124,4 (C_{3b}-C_{6c}) ; 127,7-128,0 (C_{3c}-C_{5c}) ; 130,7 (C_{quatenaire}) ; 133,7-133,8-134,4-134,9-136,0 (C_{5b}-2*C₂ₑ-C_{quaternaire}) ; 139,8 (C_{quaternaire}) ; 148,6 (C_{quaternaire}) ; 153,9-154,4-155,1-155,2-156,0 (C_{quatrnaire}, 3*CO_{carbamate}, 4*C_{phénol}) ; 186,0-186,2 (4*CO_{quinone}) ; 213,8 (2*CO_{cétone})
**SM (ESI) :** [M+Na]⁺ m/z = 1710,49
**SMHR (ESI) :** C₈₁H₈₂N₄O₃₆Na [M+Na]⁺ m/z théorique : 1709,4601 m/z trouvé : 1709,4601
C₈₁H₈₂N₄O₃₆Na₂ [M+2Na]⁺⁺ m/z théorique : 866,22466 m/z trouvé : 866,222 (z=2)

### Préparation du composé 221 :

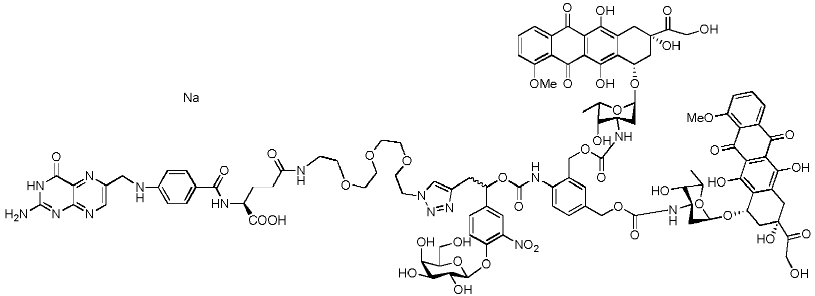

Le composé **225** (30,0 mg, 17,7 µmol) est dissous dans un mélange de 700 µl de DMSO. Le composé folate **223** (11,4 mg, 17,7 µmol), le sulfate de cuivre (2,2 mg, 8,9 µmol, 0,5 éq.) et 300 mL d'une solution d'ascorbate de sodium (3,5 mg, 17,7 µmol) dans un tampon phosphate pH 7 0,2M sont additionnés. Après une nuit d'agitation à température ambiante, le mélange réactionnel est dissous dans du méthanol puis versé sur de l'éther diéthylique refroidi. Le précipité est filtré puis décompléxé avec EDTA.2Na (41,2 mg, 4éq.) dans 1 mL d'un tampon phosphate pH 7 0,2M. Après deux heures d'agitation à température ambiante, le mélange réactionnel est dissous dans du méthanol puis versé sur de l'éther diéthylique refroidi. Après filtration, on obtient le produit **221** (11 mg, 4,7 µmol) sous forme d'une poudre rouge. Une purification par CLHP semi-préparatrice permet d'obtenir le composé **221** sous forme de 4 isomères avec une pureté de 89%.
**Formule brute :** C₁₀₈H₁₁₇N₁₅O₄₄
**M** = 2327,73 g/mol
**SMHR (ESI) :** C₁₀₈H₁₁₇N₁₅O₄₄Na [M+Na]⁺ m/z théorique : 2350,7277 m/z trouvé : 2350,7279

### Exemple 5

### Toxicité du vecteur dendritique (221)

L'efficacité du vecteur dendritique (**221**) a été comparée à celle du composé **(7)** DOX-GAL-AF et à celle de la Doxorubicine seule sur une lignée de cellules cancéreuses LAM de type KG-1.

3x10³ cellules/puits ont été mis en plaques de culture 96 puis dans 100 µl de milieu Iscove modifé Dulbecco supplémenté avec du sérum de veau foetal à 20 % (InVitrogen), et 1% pénicilline/streptomycine, à 37 °C dans une atmosphère à 5 % de CO₂. Les cellules sont cultivées pendant 24 heures avant addition des composés testés

Les cellules sont incubées en présence de concentrations croissantes (de 0 à 2000 nM) de doxorubicine (DOX), de composé (7) (DOX-GAL-AF), et de vecteur dendritique (**221**) pendant 4 jours.

Après 4 jours de traitement, la viabilité des cellules a été testé avec le kit « Cell Proliferation Kit II » (XTT ; Roche), utilisé selon les recommandations du fabricant, par addition de 50 µl d'un mélange de marquage XTT par puits. Ce test est basé sur le clivage du XTT par les cellules métaboliquement actives, se traduisant par la production d'une sonde formazan orange, qui peut être quantifiée par spectrophotométrie.

Les cellules ont été encore incubées pendant 3 h supplémentaires, à 37 °C avant mesure de l'absorbance à 480 nm. Les valeurs de CI₅₀ ont été déterminées graphiquement.

Les résultats, illustrés par la Figure 5, montrent que vecteur dendritique (**221**) (carré) réduit la viabilité des cellules LAM de type KG 1 d'un facteur d'environ 4 à 5 fois plus que le composé (7) (triangle) (0,1 µM < IC₅₀ < 0,15 µM vs IC₅₀ = 0,5 µM) (Figure 5), et est presque équivalent à celui de la Doxorubicine (rond).

Le vecteur dendritique (**221**) comporte deux molécules de doxorubicine pour un seul ligand de ciblage (acide folique). Par rapport au composé (**7**), ce vecteur peut conduire à la libération d'une quantité deux fois plus élevée de drogue à l'intérieur des cellules cibles ou dans le microenvironnement tumoral. Ainsi, grâce à l'accroissement du ratio [nombre de drogues libérées / nombre de ligands de ciblage] le vecteur dendritique (**221**) est plus efficace sur des cellules cancéreuses exprimant le récepteur à l'acide folique. En outre, l'accroissement du ratio [nombre de drogues libérées / nombre de substrats enzymatiques] permet d'obtenir une activité cytotoxique supérieure sans modifier le nombre d'événements enzymatiques.

## Revendications

1. Composé de formule générale (I) : 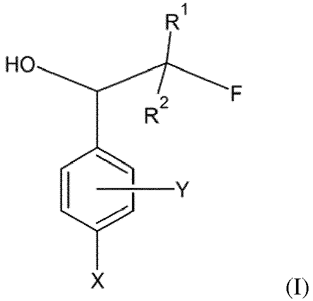 dans laquelle :
- X représente OH, NH₂, NHOH ou R'NH avec R' pouvant représenter un radical alkyle en C₁ à à C₁₀, linéaire ou ramifié, saturé ou insaturé,
- Y représente un groupe électro-attracteur choisi parmi NO₂, CF₃ ou un halogène,
- R¹ et R² représentent, indépendamment l'un de l'autre, H ou un radical alkyle en C₁ à C₁₀, linéaire ou ramifié, saturé ou insaturé,
- F représente une fonction réactive activable par chimie click.

2. Composé selon la revendication précédente, dans lequel X représente OH.

3. Composé selon la revendication 1 ou 2, dans lequel Y représente NO₂, de préférence en position *ortho* par rapport à X.

4. Composé selon l'une quelconque des revendications précédentes, dans lequel R¹ et R² représentent H.

5. Composé selon l'une quelconque des revendications précédentes, dans lequel F représente -C=CR"', -N₃, -SH, -C=CH₂, cyclooctynes, maléimide, -SO₂N₃, ou -COSR"', avec R"' représentant H ou un radical alkyle, linéaire ou ramifié, saturé ou insaturé, en C₁ à à C₁₀.

6. Composé de formule générale (II) : 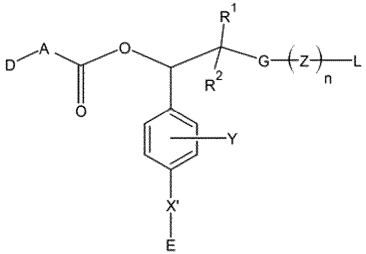 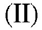 dans laquelle :
- R¹ et R² sont tels que définis en revendications 1 à 5,
- Y représente H, ou un groupe électro-attracteur, en particulier choisi parmi NO₂, CF₃ ou un halogène,
- X' représente O, NH, NOH, R'N avec R' étant tel que défini en revendication 1,
- E représente un groupe labile substrat d'une enzyme choisie parmi les glucuronidases, les glycosidases, les protéases, les peptidases, et les métalloprotéases,
- A représente O, S, NH, NR" avec R" représentant un groupe alkyle en C₁ à C₁₀, linéaire ou ramifié, saturé ou insaturé, et de préférence peut représenter NH,
- D représente un composé actif utilisable en thérapeutique ou en diagnostique,
- n = 0 ou 1, et Z peut représenter un groupe alkylène, linéaire ou ramifié, saturé ou insaturé, en C₁-C₁₀, optionnellement interrompu par un ou plusieurs hétéroatomes choisis parmi O ou N, un groupe glycosyle, un groupe O-(CHR³-CHR⁴-O-)ₘ ou N-(CHR³-CHR⁴-O-)ₘ dans lesquels m figure un entier naturel variant de 1 à 20, R³ et R⁴ figurent, indépendamment l'un de l'autre, H ou CH₃, sous réserve que R³ et R⁴ ne figurent pas simultanément CH₃, un groupe issu d'un acide aminé ou d'un peptide, ou une combinaison de ces groupes,
- L représente un ligand de ciblage choisi parmi un peptide, une protéine, un anticorps ou un fragment d'anticorps reconnaissant un antigène, un ligand d'un récepteur cellulaire, un biopolymère, un monosaccharide, un oligosaccharide, une hormone, une vitamine, un dendrimère, une polyamine, ou une nanoparticule,
- G représente un groupe résultant d'une réaction de chimie click entre un groupe F, tel que défini en revendication 1 et un groupe x-(Z)ₙ-L dans lequel Z et L sont tels que définis ci-dessus et x représente une fonction réactive activable par chimie click apte à réagir avec F,
ou un de ses sels pharmaceutiquement acceptables.

7. Composé selon la revendication 6, dans lequel Y représente NO₂ en position *ortho* de X', et R¹ et R² représentent H.

8. Composé selon l'une quelconque des revendications 6 ou 7, dans lequel le groupe labile E est un substrat de la β-glucuronidase.

9. Procédé de préparation d'un composé tel que défini selon l'une quelconque des revendications 6 à 8, consistant à faire réagir un composé de formule générale (I) : 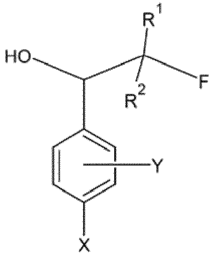 dans laquelle :
- X représente OH, NH₂, NHOH ou R'NH avec R' pouvant représenter un radical alkyle en C₁ à à C₁₀, linéaire ou ramifié, saturé ou insaturé,
- Y représente H, ou un groupe électro-attracteur, en particulier choisi parmi NO₂, CF₃ ou un halogène,
- R¹ et R² représentent H ou un radical alkyle en C₁ à C₁₀, linéaire ou ramifié, saturé ou insaturé,
- F représente une fonction réactive activable par chimie click,
avec des groupes D-v, E-w, et L-(Z)ₙ-x, dans lesquels D, E et L et Z sont tels que définis en revendication 6 ou 8, et v, w et x figurent chacun une fonction réactive telle que, à l'égard du composé de formule générale (I), v réagit avec OH lié au carbone benzylique, ou ledit OH préalablement activé, w réagit X, et x réagit avec F.

10. Procédé selon la revendication 9, dans lequel v est choisi parmi NH₂, NHR", OH, ou SH, avec R" étant tel que défini en revendication 6.

11. Procédé selon la revendication 9 ou 10, dans lequel w est choisi parmi un radical halogéné, notamment Cl ou Br, et en particulier Br, -COOH ou -OC(O)Cl.

12. Procédé selon l'une quelconque des revendications 9 à 11 dans lequel x et F sont choisis parmi les couples de fonctions réactives activables en chimie click suivants, (-N₃, -C≡CR"'), (-SH, -C=CH₂), (-N₃, cyclooctynes), (-SH, maléimide), (-SO₂N₃, -COSR"'), avec R"' représentant H ou un radical alkyle, linéaire ou ramifié, saturé ou insaturé, en C₁ à C₁₀, et notamment en C₂ à C₆, voire en C₃ ou C₄.

13. Composé ou un de ses sels pharmaceutiquement acceptables, susceptible d'être obtenu selon le procédé tel que défini selon l'une quelconque des revendications 9 à 12.

14. Composition pharmaceutique ou diagnostique comprenant au moins une quantité efficace d'au moins un composé de formule générale (II) tel que défini selon l'une quelconque des revendications 6 à 8 et 13, ou un de ses sels pharmaceutiquement acceptable.

15. Kit pour la préparation d'un composé de formule générale (II) tel que défini selon l'une quelconque des revendications 6 à 8 et 13, ou un de ses sels pharmaceutiquement acceptables, comprenant au moins un composé de formule générale (I): 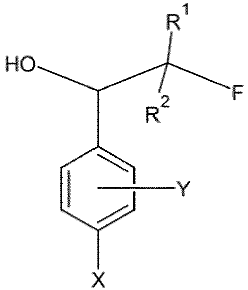 dans laquelle :
- X représente OH, NH₂, NHOH ou R'NH avec R' pouvant représenter un radical alkyle en C₁ à à C₁₀, linéaire ou ramifié, saturé ou insaturé,
- Y représente H, ou un groupe électro-attracteur, en particulier choisi parmi NO₂, CF₃ ou un halogène,
- R¹ et R² représentent H ou un radical alkyle en C₁ à C₁₀, linéaire ou ramifié, saturé ou insaturé,
- F représente une fonction réactive activable par chimie click,
et au moins un groupe choisi parmi D-v, E-w et L-(Z)ₙ-x, dans lesquels L, Z, n, D et E sont tels que définis en revendications 6 à 8 et v, w et x sont tels que définis en revendications 9 à 12, le composé de formule générale (I) et le groupe choisi parmi D-v, E-w et L-(Z)ₙ-x étant conditionné séparément.

## Patentansprüche

1. Verbindung der allgemeinen Formel (I): 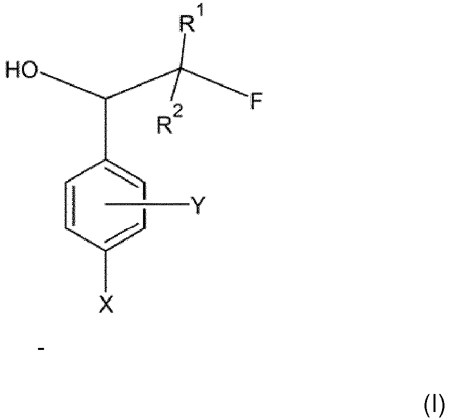 wobei:
- X OH, NH₂, NHOH oder R'NH darstellt, wobei R' einen linearen oder verzweigten, gesättigten oder ungesättigten C₁-C₁₀-Alkylrest darstellen kann,
- Y eine elektronenanziehende Gruppe darstellt, die aus NO₂, CF₃ oder einem Halogen ausgewählt ist,
- R¹ und R² unabhängig voneinander H oder einen linearen oder verzweigten, gesättigten oder ungesättigten C₁-C₁₀-Alkylrest darstellen,
- F eine reaktive Funktion darstellt, die durch Click-Chemie aktivierbar ist.

2. Verbindung gemäß dem vorhergehenden Anspruch, wobei X OH darstellt.

3. Verbindung gemäß Anspruch 1 oder 2, wobei Y NO₂, vorzugsweise bezüglich X in *ortho*-Stellung darstellt.

4. Verbindung gemäß irgendeinem der vorhergehenden Ansprüche, wobei R¹ und R² H darstellen.

5. Verbindung gemäß irgendeinem der vorhergehenden Ansprüche, wobei F -C≡CR"', -N₃, -SH, -C=CH₂, Cyclooctyne, Maleimid, -SO₂N₃ oder -COSR"' darstellt, wobei R"' H oder einen linearen oder verzweigten, gesättigten oder ungesättigten C₁-C₁₀-Alkylrest darstellt.

6. Verbindung der allgemeinen Formel (II): 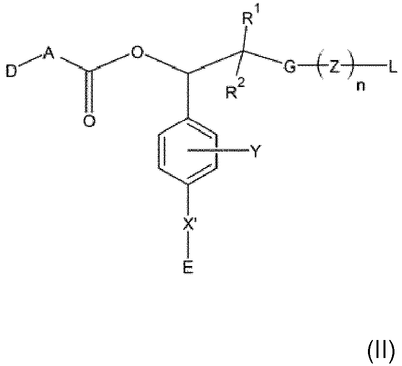 wobei:
- R¹ und R² wie in den Ansprüchen 1 bis 5 definiert sind,
- Y H oder eine elektronenanziehende Gruppe darstellt, die insbesondere aus NO₂, CF₃ oder einem Halogen ausgewählt ist,
- X' O, NH, NOH, R'N darstellt, wobei R' wie in Anspruch 1 definiert ist,
- E eine labile Substratgruppe eines Enzyms darstellt, das aus den Glucoronidasen, den Glycosidasen, den Proteasen, den Peptidasen und den Metalloproteasen ausgewählt ist,
- A O, S, NH, NR" darstellt, wobei R" eine lineare oder verzweigte, gesättigte oder ungesättigte C₁-C₁₀-Alkylgruppe darstellt, und vorzugsweise NH darstellen kann,
- D eine aktive Verbindung darstellt, die therapeutisch oder diagnostisch verwendbar ist,
- n = 0 oder 1, und Z eine lineare oder verzweigte, gesättigte oder ungesättigte C₁-C₁₀-Alkylengruppe, die optional durch ein oder mehrere Heteroatome unterbrochen ist, die aus O oder N ausgewählt sind, eine Glycosylgruppe, eine O-(CHR³-CHR⁴-O-)ₘ oder N-(CHR³-CHR⁴-O-)ₘ-Gruppe, in denen m eine natürliche Zahl darstellt, die von 1 bis 20 variiert, R³ und R⁴ unabhängig voneinander H oder CH₃ darstellen, unter der Bedingung, dass R³ und R⁴ nicht gleichzeitig CH₃ darstellen, eine aus einer Aminosäure oder einem Peptid stammende Gruppe oder eine Kombination dieser Gruppen darstellen kann,
- L einen Targeting-Liganden darstellt, der aus einem Peptid, einem Protein, einem Antikörper oder einem Antikörperfragment, welches ein Antigen erkennt, einem Liganden eines zellulären Rezeptors, einem Biopolymer, einem Monosaccharid, einem Oligosaccharid, einem Hormon, einem Vitamin, einem Dendrimer, einem Polyamin oder einem Nanopartikel ausgewählt ist,
- G eine Gruppe darstellt, die sich aus einer Click-Chemie-Reaktion zwischen einer F-Gruppe, wie in Anspruch 1 definiert, und einer x-(Z)ₙ-L-Gruppe ergibt, wobei Z und L wie oben definiert sind und x eine durch Click-Chemie aktivierbare reaktive Funktion darstellt, die in der Lage ist, mit F zu reagieren,
oder eines ihrer pharmazeutisch akzeptablen Salze.

7. Verbindung gemäß Anspruch 6, wobei Y NO₂ in *ortho*-Stellung zu X' darstellt und R¹ und R² H darstellen.

8. Verbindung gemäß irgendeinem der Ansprüche 6 oder 7, wobei die labile E-Gruppe ein Substrat der β-Glucoronidase ist.

9. Verfahren zum Herstellen einer gemäß irgendeinem der Ansprüche 6 bis 8 definierten Verbindung, das daraus besteht, eine Verbindung der allgemeinen Formel (I): 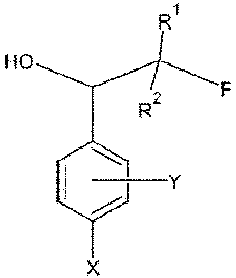 wobei:
- X OH, NH₂, NHOH oder R'NH darstellt, wobei R' einen linearen oder verzweigten, gesättigten oder ungesättigten C₁-C₁₀-Alkylrest darstellen kann,
- Y H oder eine elektronenanziehende Gruppe darstellt, die insbesondere aus NO₂, CF₃ oder einem Halogen ausgewählt wird,
- R¹ und R² H oder einen linearen oder verzweigten, gesättigten oder ungesättigten C₁-C₁₀-Alkylrest darstellen,
- F eine reaktive Funktion darstellt, die durch Click-Chemie aktivierbar ist,
mit D-v-, E-w- und L-(Z)ₙ-x-Gruppen reagieren zu lassen, wobei D, E und L und Z wie in Anspruch 6 oder 8 definiert sind und v, w und x jeweils eine reaktive Funktion darstellen, so dass, gegenüber der Verbindung der allgemeinen Formel (I), v mit OH, das an den benzylischen Kohlenstoff gebunden ist, oder dem zuvor aktivierten OH reagiert, w mit X reagiert und x mit F reagiert.

10. Verfahren gemäß Anspruch 9, wobei v aus NH₂, NHR", OH oder SH ausgewählt wird, wobei R" wie in Anspruch 6 definiert ist.

11. Verfahren gemäß Anspruch 9 oder 10, wobei w aus einem halogenierten Rest, insbesondere Cl oder Br und im Besonderen Br, -COOH oder -OC(O)Cl ausgewählt wird.

12. Verfahren gemäß irgendeinem der Ansprüche 9 bis 11, wobei x und F aus den folgenden durch Click-Chemie aktivierbaren reaktiven Funktionspaaren ausgewählt werden: (-N₃, -C≡CR"'), (-SH, -C=CH₂), (-N₃, Cyclooctyne), (-SH, Maleimid), (-SO₂N₃, -COSR"'), wobei R"' H oder einen linearen oder verzweigten, gesättigten oder ungesättigten C₁-C₁₀- und insbesondere C₂-C₆ oder sogar C₃- oder C₄-Alkylrest darstellt.

13. Verbindung oder eines ihrer pharmazeutisch akzeptablen Salze, die geeignet ist, gemäß dem Verfahren wie in irgendeinem der Ansprüche 9 bis 12 definiert erhalten zu werden.

14. Pharmazeutische oder diagnostische Zusammensetzung, aufweisend mindestens eine wirksame Menge mindestens einer Verbindung der allgemeinen Formel (II) wie gemäß irgendeinem der Ansprüche 6 bis 8 und 13 definiert, oder eines ihrer pharmazeutisch akzeptablen Salze.

15. Kit zur Herstellung einer Verbindung der allgemeinen Formel (II) wie gemäß irgendeinem der Ansprüche 6 bis 8 und 13 definiert, oder eines ihrer pharmazeutisch akzeptablen Salze, aufweisend mindestens eine Verbindung der allgemeinen Formel (I): 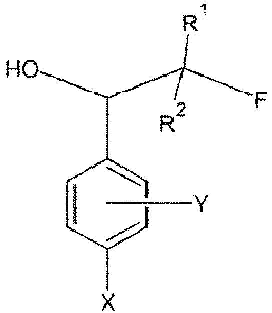 wobei:
- X OH, NH₂, NHOH oder R'NH darstellt, wobei R' einen linearen oder verzweigten, gesättigten oder ungesättigten C₁-C₁₀-Alkylrest darstellen kann,
- Y H oder eine elektronenanziehende Gruppe darstellt, die insbesondere aus NO₂, CF₃ oder einem Halogen ausgewählt ist,
- R¹ und R² H oder einen linearen oder verzweigten, gesättigten oder ungesättigten C₁-C₁₀-Alkylrest darstellen,
- F eine reaktive Funktion darstellt, die durch Click-Chemie aktivierbar ist,
und mindestens eine Gruppe, die aus D-v, E-w und L-(Z)ₙ-x ausgewählt ist, wobei L, Z, n, D und E wie in den Ansprüchen 6 bis 8 definiert sind und v, w und x wie in den Ansprüchen 9 bis 12 definiert sind, wobei die Verbindung der allgemeinen Formel (I) und die aus D-v, E-w und L-(Z)ₙ-x ausgewählte Gruppe getrennt konditioniert sind.

## Claims

1. Compound of general formula (1): 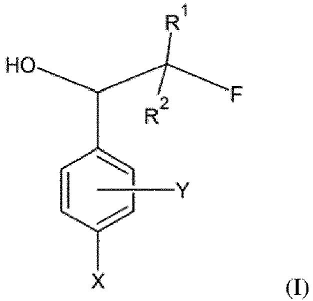 wherein:
- X represents OH, NH₂, NHOH or R'NH and R' represents a linear or branched, saturated or unsaturated C₁-C₁₀ alkyl radical,
- Y represents an electron-withdrawing group chosen from among NO₂, CF₃ or a halogen,
- R¹ and R² represent, independently of one another, H or a linear or branched, saturated or unsaturated C₁-C₁₀ alkyl radical,
- F represents a reactive function activatable by click chemistry.

2. Compound according to the preceding claim, wherein X represents OH.

3. Compound according to claim 1 or 2 wherein y represents NO₂, preferably in ortho position to X.

4. Compound according to any one of the preceding claims wherein R¹ and R² represent H.

5. Compound according to any one of the preceding claims, wherein F represents - C≡CR"', -N3, -SH, -C=CH₂, cyclooctynes, maleimide, -SO₂N₃, or -COSR"', where R"' represents H or an alkyl radical, linear or branched, saturated or unsaturated, in C₁-C₁₀.

6. Compound of general formula (II): 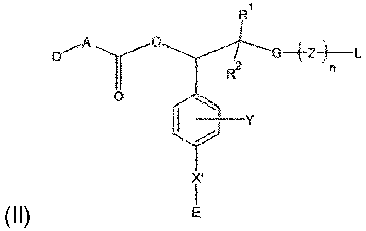 wherein:
- R¹ and R² are as defined in claims 1 to 5,
- Y represents H, or an electron-withdrawing group, in particular chosen from among NO₂, CF₃ or a halogen,
- X' represents O, NH, NOH, R'N where R' is as defined in claim 1,
- E represents a labile substrate group of an enzyme chosen from among glucuronidases, glycosidases, proteases, peptidases, and metalloproteases,
- A represents O, S, NH, NR" where R" represents a linear or branched, saturated or unsaturated C₁-C₁₀ alkyl group, and may preferably represent NH,
- D represents an active compound that may be used in therapy or in diagnostics,
- n = 0 or 1, and Z may represent a linear or branched, saturated or unsaturated, in C₁-C₁₀ alkylene group, optionally interrupted by one or more heteroatoms chosen from among O or N, a glycosyl group, a O-(CHR³-CHR⁴-O-)ₘ or N-(CHR³-CHR⁴-O-)ₘ in which m is a natural integer ranging from 1 to 20, R³ and R⁴ represent H or CH₃ independently of one another provided that R³ and R⁴ do not simultaneously represent CH₃, a group derived from an amino acid or peptide, or a combination thereof,
- L represents a targeting ligand chosen from among a peptide, a protein, an antibody or an antibody fragment which recognizes an antigen, a ligand of a cellular receptor, a biopolymer, a monosaccharide, an oligosaccharide, a hormone, a vitamin, a dendrimer, a polyamine, or a nanoparticle,
- G represents a group resulting from a click chemistry reaction between a group F as defined in claim 1 and a group x-(Z)ₙ-L wherein Z and L are as defined above, and x represents a reactive function activatable by click chemistry able to react with F, or a pharmaceutically-acceptable salt thereof.

7. Compound of claim 6, wherein Y represents NO₂ in the ortho position of X', and R¹ and R² are H.

8. Compound according to any one of the claims 6 or 7, wherein the labile group E is a β-glucuronidase, substrate.

9. Method for preparing a compound as defined according to any one of the claims 6 to 8, comprising reacting a compound of general formula (1): 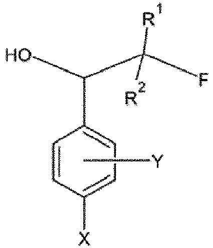 wherein:
- X represents OH, NH₂, NHOH or R'NH where R' may represent a linear or branched, saturated or unsaturated C₁-C₁₀ alkyl radical,
- Y represents H, or an electron-withdrawing group, in particular chosen from among NO₂, CF₃ or a halogen,
- R¹ and R² represent H or a linear or branched, saturated or unsaturated C₁-C₁₀ alkyl radical,
- F represents a reactive function activatable by click chemistry, with groups D-v, E-w, and L-(Z)ₙ-x, wherein D, E and L and Z are as defined in claim 6 or 8, and wherein v, w and x each has a reactive function so that, with respect to the compound of general formula (1), v is reacted with OH bonded to benzyl carbon or the OH previously activated, w reacts X, and x reacts with F.

10. Method according to claim 9, wherein v is selected from NH₂, NHR", OH, or SH,
where R" is as defined in claim 6.

11. Method according to claim 9 or 10, wherein w is selected from among a halogen radical, especially Cl or Br, and in particular Br, -COOH or -OC(O)Cl.

12. Method according to any one of the claims 9 to 11 wherein x and F are selected from among the following reactive function couples activatable by click chemistry, (-N₃, - C≡CR"'), (-SH, -C=CH₂), (-N₃, cyclooctynes), (-SH, maleimide), (-SO₂N₃, -COSR"'), where R"' represents H or a linear or branched, saturated or unsaturated in C₁ to C₁₀, especially C₂ to C₆, or even C₃ or C₄.

13. Compound or a pharmaceutically-acceptable salt thereof, obtainable by the method as defined in any one of the claims 9 to 12.

14. Pharmaceutical or diagnostic composition comprising at least an effective amount of at least one compound of general formula (II) as defined in any one of claims 6 to 8 and 13, or a pharmaceutically-acceptable salt thereof.

15. Kit for the preparation of a compound of general formula (II) as defined according to any one of the claims 6 to 8 and 13, or a pharmaceutically-acceptable salt thereof, comprising at least one compound of general formula (1): wherein: 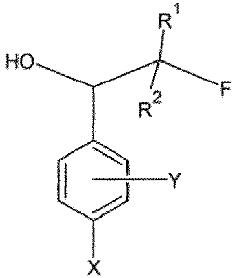
- X represents OH, NH₂, NHOH or R'NH where R' is able to represent a linear or branched, saturated or unsaturated C₁-C₁₀ alkyl radical,
- Y represents H, or an electron-withdrawing group, in particular chosen from NO₂, CF₃ or a halogen,
- R¹ and R² represent H or a linear or branched, saturated or unsaturated C₁-C₁₀ alkyl radical,
- F represents a reactive function activatable by click chemistry, and at least one group chosen from among D-v, E-w and L-(Z)ₙ-x, wherein L, Z, n, D and E are as defined in claims 6 to 8 and v, w and x are as defined in claims 9 to 12, wherein the compound of general formula (1) and the group selected from among D-v, E-w and L-(Z)ₙ-x is separately conditioned.
